# EUROPEAN PATENT APPLICATION

(11) **EP 4 763 209 A1**
(43) Date of publication of application: **24.06.2026**
(21) Application number: 24307144.6
(22) Date of filing: 17.12.2024
(51) Int. Cl.: A61K 31/7088, A61K 38/02, A61K 45/00, A61N 5/10, A61P 31/00, A61P 35/00, A61P 35/04, C07K 2/00, C07K 14/00, C12N 15/11, C12N 15/113

(54) **USE OF (ALPHA)S-SETMAR AGONIST FOR PREVENTING OR TREATING GLIOBLASTOMA**

(71) Applicant: Institut National de la Santé et de la Recherche Médicale, 75013 Paris (FR); Université de Tours, 37000 Tours (FR)
(72) Inventor: The designation of the inventor has not yet been filed
(74) Representative: Inserm Transfert

(57) **Abstract**

Inventors determined that recombinant glioblastoma cell lines overexpressing αS-SETMAR (a stable form of S-SETMAR) exhibited a prolonged cell cycle from 27 to 37 hours that leads to a reduction in cell proliferation. The over-expression of αS-SETMAR revealed an unexpected role for this protein as it increased DNA quantity linked to chromosomal chaos, both in terms of chromosomes number (aneuploidy) and organization (chromoanagenesis). Finally, they have noted that αS-SETMAR over-expression makes cells more sensitive to stringent conditions for their survival, such as those used during chemotherapy or radiotherapy treatments. These data allow them to conclude that αS-SETMAR reduces cell proliferation, sensitizes cells to chromosomal chaos and irradiation-induced apoptosis, opening up the possibility of using αS-SETMAR as a therapeutic or prognosis protein.

The present invention relates to a method for sensitizing glioblastoma cells to a classical treatment comprising a step of administrating an agonist of αS-SETMAR in a subject in need thereof.

## Description

### FIELD OF THE INVENTION:

The invention is in the field of oncology. More particularly, the invention relates to use of αS-SETMAR agonist for preventing and/or treating glioblastoma.

### BACKGROUND OF THE INVENTION:

Glioblastoma (GB, World Health Organization grade IV gliomas) are the most common primitive malignant tumour of the central nervous system, and remain one of the deadliest human cancers [1]. Despite aggressive treatments, patients with GB have 5.5% of 5-years-survival and a median survival of only 15 months. In addition, GB display a striking heterogeneity, both at cellular and morphological scale [2]. Thus, the treatment of GB is still a big challenge and this field is worth investigating.

In a previous study, inventors demonstrated that S-SETMAR, when enriched in tissues surrounding GB, correlates with an increased patients survival [3]. Born 45 million years ago, SETMAR is a fusion gene only present in higher primates. It is made of three exons, the two first given by the SET gene and coding for methyltransferase functions, the third given by the Hsmar1 transposase gene and coding for recombinase functions. The full length SETMAR protein (FL-SETMAR) is described as a genome keeper, expressed in main tissues, but with different levels. In cancer cells, the SETMAR gene is over-expressed, and FL-SETMAR sustains oncogenic processes, probably through its involvement in DNA repair by Non-Homologous End-Joining (NHEJ), replication stress response and chromosome decatenation (review in [4]). The role of FL-SETMAR in cell proliferation is more ambiguous since some studies show a positive correlation [5-7] while another suggests the contrary [8].

SETMAR pre-mRNA can undergo alternative splicing, leading to the production of shorter proteins, enriched in cancer stem cells. One of them, S-SETMAR, was first discovered in glioblastoma (GB)[9], and more recently in colorectal cancers [10]. In addition, mRNA coding for shorter variants have been detected in bladder cancer cells [11] and leukemia [12], but the corresponding proteins have not been searched.

S-SETMAR lacks a part of the pre-SET domain and the whole SET domain, both encoded by exon 2. As a result, S-SETMAR is unable to methylate proteins, as does FL-SETMAR with a moderate efficiency [13]. On the other hand, it is less efficient than FL-SETMAR in promoting NHEJ and appears to be preponderant over FL-SETMAR in GB stem cells [9]. Nothing is known about S-SETMAR mechanisms of action, or the rules that govern its production, i.e. what favors the exclusion of exon 2 during pre-mRNAs maturation.

Interestingly, S-SETMAR is a more stable protein than FL-SETMAR, as it more often carries an N-terminal sequence of 13 amino-acids called α -peptide [3]. This suggests a link between exon 2 exclusion (during alternative splicing) and the AUG codon that will be selected for the initiation of the translation. About the mechanism of action of S-SETMAR, a hypothesis formulated by several authors proposes that it could act as a dominant-negative of FL-SETMAR, by competing with partner-proteins, with DNA binding sites, and/or by poisoning FL-SETMAR through heterodimers assembly. However, the occurrence of such heterodimers has never been proved although their presence could explain some of the ambiguous results described hereafter for FL-SETMAR. Under this point of view, taking into account the amount of both proteins within cells seems a reasonable precaution when studying their mechanisms of action.

Despite many attempts to answer that question, the role of FL-SETMAR in cell proliferation remains ambiguous. It should be noted that each reported assay was very different in design. In three cases, SETMAR involvement in cell proliferation was assayed by endogenous depletion using sh- or si-RNA targeting the MAR domain, i.e. depleting cells in both SETMAR variants, leading to a decrease in cell proliferation [5-7]. Interestingly, one publication [6] described that recombinant FL-SETMAR overexpression increased cell proliferation, in a context where the cell line used did not express endogenous SETMAR (HEK-293T). Finally, a more recent study suggested that FL-SETMAR overexpression did not promote U2OS cell proliferation [8]. On the contrary, cell proliferation decreased correlated to the amount of FL-SETMAR. The same effect was observed with the overexpression of the SET domain alone. However, the endogenous level of SETMAR(s) in these cells was not shown, regardless of the variant considered. Interestingly, FL-SETMAR seemed to be missing in colorectal healthy or tumoral cells, whereas shorter variants harboring the MAR domain alone have been abundantly detected [10].

The genomic impact of SETMAR variants has been further analyzed using two approaches: ChIP-seq analyses, to find out FL-SETMAR genomic binding sites and RNA-seq to identify SETMAR-deregulated genes. Unfortunately, the four studies published so far used neither the same cell lines nor the same experimental approaches, producing results that are difficult to compare. The first [10] has shown that endogenous S-SETMAR could play a role in transcriptional regulation but also in DNA replication [7,14], via its binding to a consensus sequence of 12 base pairs, corresponding to the core sequence of TIRs (the Hsmar1 Terminal Inverted Repeats used for transposition) present in enhancers and in constitutive replication origins. The second found that over-expressed FL-SETMAR modified the expression of about 1 500 genes, mainly involved in the KEGG Pathways in Cancer. Among them only 10 % contained an Hsmar1 TIR. Interestingly, some of the non-TIR bounded sequences corresponded to a CENP-B-like binding site, usually found in centromeres. In a third study, FL-SETMAR over-expression was induced after SETMAR KO [15]. The authors have found that FL-SETMAR only bound to TIRs correlated with a strong repression of SETMAR-bound genes, the latter being involved in cell cycle control. The fourth and last study [16] confirmed the DNA binding specificity of over-expressed FL-SETMAR to TIRs sequences. In addition, transcriptomic effects of the loss of both endogenous variants (FL and S-SETMAR) have shown that 600 to 700 genes were deregulated, some of these being associated to modified expression and alternative splicing of genes associated with transcription and neuronal functions.

In conclusion, it has been previously demonstrated that FL-SETMAR binds to DNA preferentially on Hsmar1 TIRs, or similar sequences, to act directly on target-genes transcription, and even interferes with the splicing of their pre-mRNAs. Transcriptomic effects of FL-SETMAR concern genes involved in neuronal development, cell cycle control, and alternative splicing. About the epigenetics effects of FL-SETMAR, results were less consistent, the protein displaying clear non-histone lysine methyltransferase activities, but data about H3K36me2 remained divergent. Whatever the epigenetic properties of FL-SETMAR, its transcriptional activity, via the Hsmar1 TIRs network, has now been established. Pleiotropy and understanding the fine-tuning of its activities remains a challenge that probably needs to be defined by cell type, especially in brain cells, since all studies agree that SETMAR plays an essential role in the development of the primate brain.

### SUMMARY OF THE INVENTION:

The invention relates to a method for sensitizing glioblastoma cells to a classical treatment comprising a step of administrating αS-SETMAR in a subject in need thereof.

In particular, the invention is defined by the claims.

### DETAILED DESCRIPTION OF THE INVENTION:

Here, inventors' aim is to better understand the roles and mechanisms of action of S-SETMAR in GB cells. They determined that recombinant glioblastoma cell lines overexpressing αS-SETMAR (a stable form of S-SETMAR) exhibited a prolonged cell cycle from 27 to 37 hours that leads to a reduction in cell proliferation. The over-expression of αS-SETMAR revealed an unexpected role for this protein as it increased DNA quantity linked to chromosomal chaos, both in terms of chromosomes number (aneuploidy) and organization (chromoanagenesis). Finally, they have noted that αS-SETMAR over-expression makes cells more sensitive to stringent conditions for their survival, such as those used during chemotherapy or radiotherapy treatments. These data allow them to conclude that αS-SETMAR reduces cell proliferation, sensitizes cells to chromosomal chaos and irradiation-induced apoptosis, opening up the possibility of using αS-SETMAR as a therapeutic or prognosis protein.

Accordingly, **in a first aspect,** the invention relates to a method for sensitizing glioblastoma cells to a classical treatment comprising a step of administrating an agonist of αS-SETMAR in a subject in need thereof.

In a particular embodiment, the invention relates to a method for enhancing the efficacy of a classical treatment comprising a step of administrating αS-SETMAR in a subject in need thereof.

As used herein, the term **"subject"** denotes a mammal, such as a rodent, a feline, a canine, and a primate. Particularly, the subject according to the invention is a human.

In a particular embodiment, the subject is suffering or susceptible to suffering from a cancer.

In a particular embodiment, the subject is suffering or susceptible to suffering from glioblastoma.

As used herein, the term **"cancer"** refers to a malignant growth or tumour resulting from an uncontrolled division of cells. The term "cancer" includes primary tumors and metastatic tumors

In a particular embodiment, the cancer is resistant or metastatic cancer.

As used herein, the term **"resistant cancer"** also called as **"metastatic cancer"** refers to a cancer which does not respond to a treatment. The cancer may be resistant at the beginning of treatment, or it may become resistant during treatment. The resistance to drug leads to rapid progression of metastatic cancer (recurrence). In the context of the invention, recurrence may occur at the resection site or through migration of cancer cells elsewhere in the brain.

As used herein, the term **"glioblastoma"** (GB) also called glioblastoma multiforme or "grade IV astrocytoma" according to WHO classification, has its general meaning in the art and refers to central nervous system primary tumor derived from glial cells.

In a further embodiment, the glioblastoma is a mestastic cancer. The inevitable recurrence of GBM is associated to: (i) resistance to radio and chemo-therapy; (ii) diffuse features due to the invasiveness properties of tumor cells throughout the surrounding brain parenchyma and (iii) heterogeneity observed between GB patients but also within the same tumor.

In a particular embodiment, glioblastoma is characterized by the enrichment of presence of stem cells (GSC).

As used herein the term **"Glioblastoma Stem Cells (GSC)"** denotes a subpopulation of highly tumorigenic and radioresistant stem cells present in the brain tumor. These cells can express CD133 gene and/or a panel of other genes characteristic of neural stem cells (Nestin, A2B5, 01ig2, Sox2, etc..) and possess the self-renewal potential (see Cheng L et al. 2010).

Thus as used herein, the term "enrichment of stem cells (GSC) in glioblastoma" denotes an over-presence of GSC in glioblastoma. As used herein, the term "β8 integrin" has it general meaning in the art and refers to a member of the integrin's family which are receptors that mediate attachment between a cell and the tissues surrounding it, which may be other cells or the extracellular matrix (ECM). They also play a role in cell signaling and thereby regulate cellular shape, motility, invasion, angiogenesis, survival and the cell cycle.

As used herein, **"a tumour microenvironment"** (TME) refers to a highly complex and dynamic structure of cells of which a variety of immune cells are a major component. The TME contains cells of the immune system (e.g., T cells, B cells, dendritic cells, myeloid-derived suppressor cells (MDSCs), tumour-associated macrophages (T AMs)), a complicated network of fibroblasts, blood vessels, lymphatics, and the cells of the cancer itself. These TME elements are surrounded by a dense meshwork of collagen and elastin fibres that comprise the extracellular matrix (ECM), wherein the TME is also composed of a complicated network of cytokines, chemokines, growth factors, and inflammatory, as well as matrix remodelling enzymes. Furthermore, the TME is a critical facilitator of immune escape and cancer progression, and the interaction between cancer cells and the diverse cell population within the TME influences tumour resistance, progression, and metastasis.

As used herein, the term **"sensitizing"** or **"improve sensitivity"** refers to increasing response rates to a specific treatment (e g., chemotherapy, immunotherapy etc).

In some embodiments, improving sensitivity to ICB treatment can refer to inhibiting negative regulatory immune checkpoints or stimulating activating immune checkpoints. In some embodiments, the subject has refractory cancer (e. g. , a refractory' tumor). In some embodiments, the subject has undergone previous treatments for cancer.

In some embodiments, the subject has not shown improvement in cancer symptoms and/or cancer markers after previous treatments for cancer.

In some embodiments, administration of αS-SETMAR sensitizes a response to classical treatment such as chemotherapy or immunotherapy in a subject. Such subject shows improvement in cancer symptoms and/or cancer markers over similar classical treatment without administration of αS-SETMAR.

As used herein, the term **"classical treatment"** refers to treatments well known in the art and used to treat cancer. In the context of the invention, the classical treatment refers to the group consisting of but not limited to: radiation therapy, immunotherapy, chemotherapy (= temozolomide (TMZ)) and surgery.

In a particular embodiment, the method for sensitizing cancer cells according to the invention wherein, the classical treatment is immunotherapy.

As used herein, the term **"immunotherapy"** or **"immunotherapy treatment"** refers to a cancer therapeutic treatment using the immune system to reject cancer. The therapeutic treatment stimulates the patient's immune system to attack the malignant tumor cells. It includes immunization of the patient with tumor antigens (e.g. by administering a cancer vaccine), in which case the patient's own immune system is trained to recognize tumor cells as targets to be destroyed, or administration of molecules stimulating the immune system such as cytokines, or administration of therapeutic antibodies such as drugs, in which case the patient's immune system is recruited by the therapeutic antibodies to destroy tumor cells. In particular, antibodies are directed against specific antigens such as the unusual antigens that are presented on the surfaces of tumors. In a further embodiment, the immunotherapy refers to inhibit immune checkpoint.

As used herein, the term **"immune checkpoint"** or **"immune checkpoint protein"** has its general meaning in the art and refers to a molecule that is expressed by immune or cancer cells in that either turn up a signal (stimulatory checkpoint molecules) or turn down a signal (inhibitory checkpoint molecules). Many of the immune checkpoints are regulated by interactions between specific receptor and ligand pairs. Overexpression of inhibitory checkpoint molecules by cancer cells have often been associated with inhibition of anti tumor immune response as immune cell express their ligand or receptor counterparts.

Immune checkpoint molecules are recognized in the art to constitute immune checkpoint pathways similar to the CTLA-4 and PD-1 dependent pathways (see e.g. Pardoll, 2012. Nature Rev Cancer 12:252-264; Mellman et al. 2011. Nature 480:480- 489). Examples of stimulatory checkpoint include CD27 CD28 CD40, CD122, CD137, OX40, GITR, and ICOS. Examples of inhibitory checkpoint molecules include A2AR, B7-H3, B7-H4, BTLA, CTLA-4, CD277, IDO, KIR, PD-1, LAG-3, TIM-3 and VISTA. The Adenosine A2A receptor (A2AR) is regarded as an important checkpoint in cancer therapy because adenosine in the immune microenvironment, leading to the activation of the A2a receptor, is negative immune feedback loop and the tumor microenvironment has relatively high concentrations of adenosine. B7-H3, also called CD276, was originally understood to be a co-stimulatory molecule but is now regarded as co-inhibitory. B7-H4, also called VTCN1, is expressed by tumor cells and tumor-associated macrophages and plays a role in tumour escape. B and T Lymphocyte Attenuator (BTLA) and also called CD272, has HVEM (Herpesvirus Entry Mediator) as its ligand. Surface expression of BTLA is gradually downregulated during differentiation of human CD8+ T cells from the naive to effector cell phenotype, however tumor-specific human CD8+ T cells express high levels of BTLA. CTLA-4, Cytotoxic T-Lymphocyte-Associated protein 4 and also called CD152. Expression of CTLA-4 on Treg cells serves to control T cell proliferation. IDO, Indoleamine 2,3-dioxygenase, is a tryptophan catabolic enzyme. A related immune-inhibitory enzymes. Another important molecule is TDO, tryptophan 2,3-dioxygenase. IDO is known to suppress T and NK cells, generate and activate Tregs and myeloid-derived suppressor cells, and promote tumour angiogenesis. KIR, Killer-cell Immunoglobulin-like Receptor, is a receptor for MHC Class I molecules on Natural Killer cells. LAG3, Lymphocyte Activation Gene-3, works to suppress an immune response by action to Tregs as well as direct effects on CD8+ T cells. PD-1, Programmed Death 1 (PD-1) receptor, has two ligands, PD-L1 and PD-L2. This checkpoint is the target of Merck & Co.'s melanoma drug Keytruda, which gained FDA approval in September 2014. An advantage of targeting PD-1 is that it can restore immune function in the tumor microenvironment. TIM-3, short for T-cell Immunoglobulin domain and Mucin domain 3, expresses on activated human CD4+ T cells and regulates Th1 and Th17 cytokines. TIM-3 acts as a negative regulator of Th1/Tc1 function by triggering cell death upon interaction with its ligand, galectin-9. VISTA, Short for V-domain Ig suppressor of T cell activation, VISTA is primarily expressed on hematopoietic cells so that consistent expression of VISTA on leukocytes within tumors may allow VISTA blockade to be effective across a broad range of solid tumors. Tumor cells often take advantage of these checkpoints to escape detection by the immune system. Thus, inhibiting a checkpoint protein on the immune system may enhance the anti-tumor T-cell response.

As used herein, the expressions **"immune checkpoint inhibitor", "checkpoint inhibitor"** or **"checkpoint blockade cancer immunotherapy agent"** are used interchangeably and have its general meaning in the art and refers to any compound inhibiting the function of an immune checkpoint protein. Inhibition includes reduction of function and full blockade. The immune checkpoint inhibitors include peptides, proteins, antibodies, nucleic acid molecules and small molecules. Preferred immune checkpoint inhibitors are antibodies that specifically recognize immune checkpoint proteins. Examples of immune checkpoint inhibitors are provided here below under the associated paragraph.

In a particular embodiment, the immune checkpoint inhibitor is an antibody.

Typically, antibodies are directed against PD-1, CTLA-4, A2AR, B7-H3, B7-H4, BTLA, CD277, IDO, KIR, LAG-3, TIM-3 or VISTA.

In a particular embodiment, the immune checkpoint inhibitor is an anti-PD-1 antibody such as described in WO2011082400, WO2006121168, WO2015035606, WO2004056875, WO2010036959, WO2009114335, WO2010089411, WO2008156712, WO2011110621, WO2014055648 and WO2014194302. Examples of anti-PD-1 antibodies which are commercialized: Nivolumab (Opdivo^{®}, BMS), Pembrolizumab (also called Lambrolizumab, KEYTRUDA^{®} or MK-3475, MERCK).

In some embodiments, the immune checkpoint inhibitor is an anti-PD-L1 antibody such as described in WO2013079174, WO2010077634, WO2004004771, WO2014195852, WO2010036959, WO2011066389, WO2007005874, WO2015048520, US8617546 and WO2014055897. Examples of anti-PD-L1 antibodies which are on clinical trial: Atezolizumab (MPDL3280A, Genentech/Roche), Durvalumab (AZD9291, AstraZeneca), Avelumab (also known as MSB0010718C, Merck) and BMS-936559 (BMS).

In a particular embodiment, the anti-PD-1 or anti-PD-L1 antibody is atezolizumab, durvalumab, avelumab, nivolumab, pembrolizumab, pidilizumab, cemiplimab, camrelizumab, sintilimab (IBI308), tislelizumab (BGB-A317), toripalimab (JS 001), dostarlimab (TSR-042, WBP-285), BMS 936559, MPDL3280A, MSB0010718C, MEDI4736 and any combination thereof.

In a particular embodiment, the immune checkpoint inhibitor is an antibody directed against CTLA-4. Antibodies directed against CTLA-4 are also known such as ipilimumab, tremelimumab, MK-1308, AGEN-1884, XmAb20717 (Xencor), MEDI5752 (AstraZeneca).

In some embodiments, the monotherapy is performed with an-anti PD-1. More particularly, the anti-PD-1 is nivolumab.

In some embodiments, the bi-therapy (as a combined therapy) is performed with anti-PD-1 inhibitor and anti-CTLA-4 inhibitor, and in particular with anti-PD-1 antibody and anti-CTLA-4 antibody. More particularly, the anti-PD-1 is nivolumab and anti-CTLA-4 is ipilimumab.

In some embodiments, the immune checkpoint inhibitor is an anti-PD-L2 antibody such as described in US7709214, US7432059 and US8552154.

In some embodiments, the immune checkpoint inhibitor inhibits Tim-3 or its ligand.

In a particular embodiment, the immune checkpoint inhibitor is an anti-Tim-3 antibody such as described in WO03063792, WO2011155607, WO2015117002, WO2010117057 and WO2013006490.

In a particular embodiment, the immune checkpoint inhibitor is a monoclonal antibody directed against LAG-3. Antibodies directed against LAG-3 are also known such as: Relatlimab (BMS-986016), Favezelimab (MK-4280, Merck), LAG3-Ab (Protheragen), TJA-3(I-Mab), LBL-007(BeiGene), LAG525(Novartis), Tesaro (GSK) (TSR-033), Sym022 (Symphogen), GSK2831781 (GlaxoSmith), INCAGN02385 (Incyte Biosciences International), IMP321(Prima BioMed/Immutep), MGD013 (MacroGenics), FS118 (F-Star), RO7247669 (Hoffmann-La Roche), EMB-02(Shanghai EpimAb Biotherapeutics), XmAb841 (Xencor), Fianlimab (REGN3767) or IBI323(Innovent Biologics).

In a particular embodiment, the immune checkpoint inhibitor is a bispecific antibody directed against LAG-3 and another immune check point (PD-1, PDL-1, PD-2 etc). Bispecific antibodies directed against LAG-3 are also known such as: Tebotelimab (MGD013, Macrogenetics), FS118 (F-star Therapeutics), RO7247669 (Hoffmann-La Roche), EMB-02 (EpimAb Biotherapeutics).

In some embodiments, the immune checkpoint inhibitor is a small organic molecule.

The term "small organic molecule" as used herein, refers to a molecule of a size comparable to those organic molecules generally used in pharmaceuticals. The term excludes biological macro molecules (e. g. proteins, nucleic acids, etc.). Typically, small organic molecules range in size up to about 5000 Da, more preferably up to 2000 Da, and most preferably up to about 1000 Da.

Typically, the small organic molecules interfere with transduction pathway of A2AR, B7-H3, B7-H4, BTLA, CTLA-4, CD277, IDO, KIR, PD-1, LAG-3, TIM-3 or VISTA.

In a particular embodiment, small organic molecules interfere with transduction pathway of PD-1 and Tim-3. For example, they can interfere with molecules, receptors or enzymes involved in PD-1 and Tim-3 pathway.

In a particular embodiment, the small organic molecules interfere with Indoleamine-pyrrole 2,3-dioxygenase (IDO) inhibitor. IDO is involved in the tryptophan catabolism (Liu et al 2010, Vacchelli et al 2014, Zhai et al 2015). Examples of IDO inhibitors are described in WO 2014150677. Examples of IDO inhibitors include without limitation 1-methyl-tryptophan (IMT), β- (3-benzofuranyl)-alanine, β-(3-benzo(b)thienyl)-alanine), 6-nitro-tryptophan, 6-fluoro-tryptophan, 4-methyl-tryptophan, 5 -methyl tryptophan, 6-methyl-tryptophan, 5-methoxy-tryptophan, 5 -hydroxy-tryptophan, indole 3-carbinol, 3,3'- diindolylmethane, epigallocatechin gallate, 5-Br-4-Cl-indoxyl 1,3-diacetate, 9- vinylcarbazole, acemetacin, 5-bromo-tryptophan, 5-bromoindoxyl diacetate, 3- Amino-naphtoic acid, pyrrolidine dithiocarbamate, 4-phenylimidazole a brassinin derivative, a thiohydantoin derivative, a β-carboline derivative or a brassilexin derivative. In a particular embodiment, the IDO inhibitor is selected from 1-methyl-tryptophan, β-(3- benzofuranyl)-alanine, 6-nitro-L-tryptophan, 3-Amino-naphtoic acid and β-[3- benzo(b)thienyl] -alanine or a derivative or prodrug thereof.

In a particular embodiment, the inhibitor of IDO is Epacadostat, (INCB24360, INCB024360) has the following chemical formula in the art and refers to -N-(3-bromo-4-fluorophenyl)-N'-hydroxy-4-{[2-(sulfamoylamino)-ethyl]amino}-1,2,5-oxadiazole-3 carboximidamide :

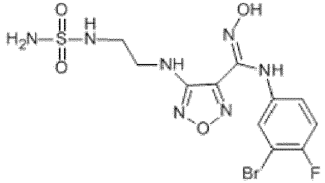

In a particular embodiment, the inhibitor is BGB324, also called R428, such as described in WO2009054864, refers to 1H-1,2,4-Triazole-3,5-diamine, 1-(6,7-dihydro-SH-benzo[6,7]cyclohepta[1,2-c]pyridazin-3-yl)-N3-[(7S)-6,7,8,9-tetrahydro-7-(1-pyrrolidinyl)-SH-benzocyclohepten-2-yl]- and has the following formula in the art:

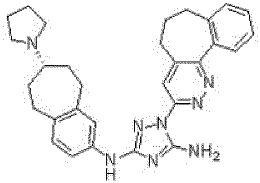

In a particular embodiment, the inhibitor is CA-170 (or AUPM-170): an oral, small molecule immune checkpoint antagonist targeting programmed death ligand-1 (PD-L1) and V-domain Ig suppressor of T cell activation (VISTA) (Liu et al 2015). Preclinical data of CA-170 are presented by Curis Collaborator and Aurigene on November at ACR-NCI-EORTC International Conference on Molecular Targets and Cancer Therapeutics.

In some embodiments, the immune checkpoint inhibitor is an aptamer.

Typically, the aptamers are directed against A2AR, B7-H3, B7-H4, BTLA, CTLA-4, CD277, IDO, KIR, PD-1, LAG-3, TIM-3 or VISTA.

In a particular embodiment, aptamers are DNA aptamers such as described in Prodeus et al 2015. A major disadvantage of aptamers as therapeutic entities is their poor pharmacokinetic profiles, as these short DNA strands are rapidly removed from circulation due to renal filtration. Thus, aptamers according to the invention are conjugated to with high molecular weight polymers such as polyethylene glycol (PEG). In a particular embodiment, the aptamer is an anti-PD-1 aptamer. Particularly, the anti-PD-1 aptamer is MP7 pegylated as described in Prodeus et al 2015.

In a particular embodiment, the method for sensitizing cancer cells according to the invention wherein, the classical treatment is radiotherapy.

As used herein, the term **"radiation therapy"** or **"radiotherapy"** have their general meaning in the art and refers the treatment of cancer with ionizing radiation. Ionizing radiation deposits energy that injures or destroys cells in the area being treated (the target tissue) by damaging their genetic material, making it impossible for these cells to continue to grow. One type of radiation therapy commonly used involves photons, e.g. X-rays. Depending on the amount of energy they possess, the rays can be used to destroy cancer cells on the surface of or deeper in the body. The higher the energy of the x-ray beam, the deeper the x-rays can go into the target tissue. Linear accelerators and betatrons produce x-rays of increasingly greater energy. The use of machines to focus radiation (such as x-rays) on a cancer site is called external beam radiation therapy. Gamma rays are another form of photons used in radiation therapy. Gamma rays are produced spontaneously as certain elements (such as radium, uranium, and cobalt 60) release radiation as they decompose, or decay. In some embodiments, the radiation therapy is external radiation therapy. Examples of external radiation therapy include, but are not limited to, conventional external beam radiation therapy; three-dimensional conformal radiation therapy (3D-CRT), which delivers shaped beams to closely fit the shape of a tumor from different directions; intensity modulated radiation therapy (IMRT), e.g., helical tomotherapy, which shapes the radiation beams to closely fit the shape of a tumor and also alters the radiation dose according to the shape of the tumor; conformal proton beam radiation therapy; image-guided radiation therapy (IGRT), which combines scanning and radiation technologies to provide real time images of a tumor to guide the radiation treatment; intraoperative radiation therapy (IORT), which delivers radiation directly to a tumor during surgery; stereotactic radiosurgery, which delivers a large, precise radiation dose to a small tumor area in a single session; hyperfractionated radiation therapy, e.g., continuous hyperfractionated accelerated radiation therapy (CHART), in which more than one treatment (fraction) of radiation therapy are given to a subject per day; and hypofractionated radiation therapy, in which larger doses of radiation therapy per fraction is given but fewer fractions.

In a particular embodiment, the method for sensitizing cancer cells according to the invention wherein, the classical treatment is chemotherapy.

As used herein, the term **"chemotherapy"** refers to use of chemotherapeutic agents to treat a subject. As used herein, the term **"chemotherapeutic agent"** refers to chemical compounds that are effective in inhibiting tumor growth.

Examples of chemotherapeutic agents include alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaorarnide and trimethylolomelamine; acetogenins (especially bullatacin and bullatacinone); a carnptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlomaphazine, cholophosphamide, estrarnustine, ifosfamide, mechlorethamine, mechlorethamine oxide hydrochloride, melphalan, novembichin, phenesterine, prednimus tine, trofosfamide, uracil mustard; nitrosureas such as carmustine, chlorozotocin, fotemustine, lomustine, nimustine, ranimustine; antibiotics such as the enediyne antibiotics (e.g. calicheamicin, especially calicheamicin (11 and calicheamicin 211, see, e.g., Agnew Chem Intl. Ed. Engl. 33: 183-186 (1994); dynemicin, including dynemicin A; an esperamicin; as well as neocarzinostatin chromophore and related chromoprotein enediyne antiobiotic chromomophores), aclacinomysins, actinomycin, authramycin, azaserine, bleomycins, cactinomycin, carabicin, canninomycin, carzinophilin, chromomycins, dactinomycin, daunorubicin, detorubicin, 6-diazo-5-oxo-L-norleucine, doxorubicin (including morpholino- doxorubicin, cyanomorpholino-doxorubicin, 2-pyrrolino-doxorubicin and deoxydoxorubicin), epirubicin, esorubicin, idanrbicin, marcellomycin, mitomycins, mycophenolic acid, nogalarnycin, olivomycins, peplomycin, potfiromycin, puromycin, quelamycin, rodorubicin, streptomgrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti- adrenals such as aminoglutethimide, mitotane, trilostane; folic acid replenisher such as frolinic acid; aceglatone; aldophospharnide glycoside; aminolevulinic acid; amsacrine; bestrabucil; bisantrene; edatraxate; defo famine; demecolcine; diaziquone; elfornithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pento statin; phenamet; pirarubicin; podophyllinic acid; 2-ethylhydrazide; procarbazine; PSK^{®}; razoxane; rhizoxin; sizofiran; spirogennanium; tenuazonic acid; triaziquone; 2,2',2"-trichlorotriethylarnine; trichothecenes (especially T-2 toxin, verracurin A, roridinA and anguidine); urethan; vindesine; dacarbazine; mannomustine; mitobromtol; mitolactol; pipobroman; gacytosine; arabinoside ("Ara-C"); cyclophosphamide; thiotepa; taxoids, e.g. paclitaxel (TAXOL^{®}, Bristol-Myers Squibb Oncology, Princeton, N.].) and doxetaxel (TAXOTERE^{®}, Rhone-Poulenc Rorer, Antony, France); chlorambucil; gemcitabine; 6-thioguanine; mercaptopurine; methotrexate; platinum analogs such as cisp latin and carbop latin; vinblastine; platinum; etoposide (VP- 16); ifosfamide; mitomycin C; mitoxantrone; vincristine; vinorelbine; navelbine; novantrone; teniposide; daunomycin; aminopterin; xeloda; ibandronate; CPT-1 1 ; topoisomerase inhibitor RFS 2000; difluoromethylomithine (DMFO); retinoic acid; capecitabine; and pharmaceutically acceptable salts, acids or derivatives of any of the above. Also included in this definition are antihormonal agents that act to regulate or inhibit honnone action on tumors such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and pharmaceutically acceptable salts, acids or derivatives of any of the above.

As used herein, the term "**SETMAR**" refers to SET Domain and Mariner Transposase fusion gene. It is made of three exons, the two first given by the SET gene and coding for methyltransferase functions, the third given by the Hsmar1 transposase gene and coding for recombinase functions. The full length SETMAR protein (FL-SETMAR) is described as a genome keeper, expressed in main tissues, but with different levels.

The naturally occurring human SETMAR gene has a nucleotide sequence as shown in Genbank Accession numbers NM_001243723, NM_001276325, NM_006515, NM_001320676, NM_001320677 ; and the naturally occurring human SETMAR protein has an aminoacid sequence as shown in Genbank Accession number NP_001230652, NP_001263254, NP_001307605, NP_001307606, NP_001307607.
The DNA nucleotide sequence of FL-SETMAR (2055 nt): SEQ ID NO: 1 
ATGTTCGCGGAAGCGGCAAAGACGACACGGCCTTGTGGGATGGCGGAGTTTAAG
GAGAAGCCTGAGGCCCCGACTGAGCAGCTGGATGTCGCGTGCGGCCAGGAAAAC
TTGCCGGTGGGCGCGTGGCCCCCGGGGGCCGCGCCGGCGCCCTTCCAGTACACTC
CTGATCATGTAGTTGGACCTGGAGCAGACATTGATCCCACTCAAATAACCTTTCC
CGGATGCATTTGTGTCAAAACTCCCTGCCTCCCTGGCACTTGCTCCTGTCTCCGCC
ATGGAGAGAACTATGATGATAACTCATGCCTTAGAGATATAGGATCTGGAGGAA
AGTATGCAGAGCCTGTTTTTGAATGCAATGTCCTGTGCCGATGCAGTGACCACTG
CAGAAACAGAGTGGTCCAGAAAGGTCTACAGTTCCACTTCCAAGTGTTCAAGACG
CATAAAAAAGGCTGGGGACTTCGTACCTTGGAATTTATACCGAAAGGAAGGTTTG
TCTGTGAATATGCTGGTGAGGTTTTAGGATTCTCTGAAGTTCAGAGAAGAATTCA
CTTACAAACAAAATCCGACTCCAATTACATTATAGCCATCAGGGAACATGTTTAT
AATGGGCAGGTAATGGAAACATTTGTTGACCCTACTTATATAGGAAATATTGGAA
GATTCCTTAATCATTCTTGTGAGCCAAACCTTTTGATGATTCCTGTCCGAATTGAC
TCAATGGTACCTAAGTTGGCACTTTTTGCAGCCAAAGATATTGTGCCAGAAGAAG
AACTCTCTTATGATTATTCAGGAAGATATCTTAATCTAACAGTCAGTGAAGACAA
AGAAAGGCTAGATCATGGGAAACTAAGGAAACCTTGTTACTGTGGTGCCAAATC
ATGTACTGCTTTCCTGCCTTTTGACAGTTCTCTGTACTGCCCCGTAGAAAAGTCGA
ACATCAGTTGTGGAAATGAGAAGGAACCCAGCATGTGTGGCTCAGCCCCTTCTGT
GTTCCCCTCCTGCAAGCGATTGACCCTTGAGACTATGAAAATGATGTTAGACAAA
AAGCAAATTCGAGCAATTTTCTTATTCGAGTTCAAAATGGGTCGTAAAGCAGCAG
AAACAACTCGCAACATCAACAATGCATTTGGCCCAGGAACTGCTAACGAACGTA
CAGTGCAGTGGTGGTTCAAGAAGTTTTGCAAAGGAGATGAGAGCCTTGAAGATG
AGGAGCGTAGTGGCCGGCCATCAGAAGTTGACAACGACCAGTTGAGAGCAATC
ATCGAAGCTGATCCCCTTACAACTACACGAGAAGTTGCTGAAGAACTCAATGTCA
ACCATTCTACGGTCGTTCGACATTTGAAGCAAATTGGAAAGGTGAAAAAGCTCGA
TAAGTGGGTGCCTCATGAGCTGACTGAAAATCAAAAAAATCGTCGTTTTGAAGTG
TCATCTTCTCTTATTCTACGCAACCACAACGAACCATTTCTCGATCGGATTGTGAC
GTGTGATGAAAAGTGGATTTTATATGACAACCGGCGACGATCAGCTCAGTGGTTG
GATCAAGAAGAAGCTCCAAAGCACTTCCCAAAGCCAATCTTGCACCCAAAAAAG
GTCATGGTCACTATTTGGTGGTCTGCTGCTGGTCTGATCCACTACAGCTTTCTGAA
TCCCGGTGAAACCATTACATCTGAGAAGTATGCTCAGGAAATCGATGAGATGAAC
CAAAAACTGCAACGCCTGCAGCTGGCATTGGTCAACAGAAAGGGCCCAATTCTTC
TCCACGACAATGCCCGACCGCATGTTGCACAACCCACACTTCAAAAGTTGAATGA
 
ATTGGGCTATGAAGTTTTGCCTCATCCACCGTATTCACCTGACCTCTTGCCAACCA
ACTACCACGTCTTTAAGCATCTCAACAACTTTTTGCAGGGAAAACGCTTCCACAA
CCAGCAGGATGCAGAAAATGCTTTCCAAGAGTTCGTCGAATCCCAAAGCACGGA
TTTTTACGCTACAGGAATAAACCAACTTATTTCTCGTTGGCAAAAATGTGTTGA
TTGTAATGGTTCCTATTTTGATTAA
The protein sequence of FL-SETMAR (684 aa): SEQ ID NO : 2 
MFAEAAKTTRPCGMAEFKEKPEAPTEQLDVACGQENLPVGAWPPGAAPAPFQYTPDHVVGP
GADIDPTQITFPGCICVKTPCLPGTCSCLRHGENYDDNSCLRDIGSGGKYAEPVFECNVLCRCS
DHCRNRVVQKGLQFHFQVFKTHKKGWGLRTLEFIPKGRFVCEYAGEVLGFSEVQRRIHLQTK
SDSNYIIAIREHVYNGQVMETFVDPTYIGNIGRFLNHSCEPNLLMIPVRIDSMVPKLALFAAKDI
VPEEELSYDYSGRYLNLTVSEDKERLDHGKLRKPCYCGAKSCTAFLPFDSSLYCPVEKSNISC
GNEKEPSMCGSAPSVFPSCKRLTLETMKMMLDKKQIRAIFLFEFKMGRKAAETTRNINNAFG
PGTANERTVQWWFKKFCKGDESLEDEERSGRPSEVDNDQLRAIIEADPLTTTREVAEELNVN
HSTVVRHLKQIGKVKKLDKWVPHELTENQKNRRFEVSSSLILRNHNEPFLDRIVTCDEKWILY
DNRRRSAQWLDQEEAPKHFPKPILHPKKVMVTIWWSAAGLIHYSFLNPGETITSEKYAQEIDE
MNQKLQRLQLALVNRKGPILLHDNARPHVAQPTLQKLNELGYEVLPHPPYSPDLLPTNYHVF
KHLNNFLQGKRFHNQQDAENAFQEFVESQSTDFYATGINQLISRWQKCVDCNGSYFD*
The DNA nucleotide sequence of αS-SETMAR (1191 nt) : SEQ ID NO : 3 
ATGTTCGCGGAAGCGGCAAAGACGACACGGCCTTGTGGGATGGCGGAGTTTAAG
GAGAAGCCTGAGGCCCCGACTGAGCAGCTGGATGTCGCGTGCGGCCAGGAAAAC
TTGCCGGTGGGCGCGTGGCCCCCGGGGGCCGCGCCGGCGCCCTTCCAGACTATGA
AAATGATGTTAGACAAAAAGCAAATTCGAGCAATTTTCTTATTCGAGTTCAAAAT
GGGTCGTAAAGCAGCAGAAACAACTCGCAACATCAACAATGCATTTGGCCCAGG
AACTGCTAACGAACGTACAGTGCAGTGGTGGTTCAAGAAGTTTTGCAAAGGAGA
TGAGAGCCTTGAAGATGAGGAGCGTAGTGGCCGGCCATCAGAAGTTGACAACGA
CCAGTTGAGAGCAATCATCGAAGCTGATCCCCTTACAACTACACGAGAAGTTGCT
GAAGAACTCAATGTCAACCATTCTACGGTCGTTCGACATTTGAAGCAAATTGGAA
AGGTGAAAAAGCTCGATAAGTGGGTGCCTCATGAGCTGACTGAAAATCAAAAAA
ATCGTCGTTTTGAAGTGTCATCTTCTCTTATTCTACGCAACCACAACGAACCATTT
CTCGATCGGATTGTGACGTGTGATGAAAAGTGGATTTTATATGACAACCGGCGAC
GATCAGCTCAGTGGTTGGATCAAGAAGAAGCTCCAAAGCACTTCCCAAAGCCAA
TCTTGCACCCAAAAAAGGTCATGGTCACTATTTGGTGGTCTGCTGCTGGTCTGATC
CACTACAGCTTTCTGAATCCCGGTGAAACCATTACATCTGAGAAGTATGCTCAGG
 
AAATCGATGAGATGAACCAAAAACTGCAACGCCTGCAGCTGGCATTGGTCAACA
GAAAGGGCCCAATTCTTCTCCACGACAATGCCCGACCGCATGTTGCACAACCCAC
ACTTCAAAAGTTGAATGAATTGGGCTATGAAGTTTTGCCTCATCCACCGTATTCAC
CTGACCTCTTGCCAACCAACTACCACGTCTTTAAGCATCTCAACAACTTTTTGCAG
GGAAAACGCTTCCACAACCAGCAGGATGCAGAAAATGCTTTCCAAGAGTTCGTC
GAATCCCAAAGCACGGATTTTTACGCTACAGGAATAAACCAACTTATTTCTCGTT
GGCAAAAATGTGTTGATTGTAATGGTTCCTATTTTGATTAA
The protein sequence of αS-SETMAR (396 aa): **SEQ ID NO : 4**

MFAEAAKTTRPCGMAEFKEKPEAPTEQLDVACGQENLPVGAWPPGAAPAPFQT
MKMMLDKKQIRAIFLFEFKMGRKAAETTRNINNAFGPGTANERTVQWWFKKFCKG
DESLEDEERSGRPSEVDNDQLRAIIEADPLTTTREVAEELNVNHSTVVRHLKQIGKVK
KLDKWVPHELTENQKNRRFEVSSSLILRNHNEPFLDRIVTCDEKWILYDNRRRSAQW
LDQEEAPKHFPKPILHPKKVMVTIWWSAAGLIHYSFLNPGETITSEKYAQEIDEMNQK
LQRLQLALVNRKGPILLHDNARPHVAQPTLQKLNELGYEVLPHPPYSPDLLPTNYHV
FKHLNNFLQGKRFHNQQDAENAFQEFVESQSTDFYATGINQLISRWQKCVDCNGSYF
D*

As used herein the term **"agonist"** refers to an agent (i.e. a peptide) for which a natural or synthetic compound has a biological effect to increase the activity of for example αS-SETMAR.

As used herein the term " **αS-SETMAR agonist"** refers to an agonist of αS-SETMAR which is a molecule that has a biological effect to increase the activity of αS-SETMAR.The biological activity of αS-SETMAR depends on the amount of the protein (i.e. its expression level) as well as on the activity of the protein. Therefore, the αS-SETMAR agonist may increase the expression or activity of αS-SETMAR protein in target cells.

In some embodiments, the treatment consists of administering to the subject a αS-SETMAR agonist.

In a particular embodiment the αS-SETMAR agonist is a polypeptide selected from the group consisting of a dominant activated mutant of αS-SETMAR, a wild-type αS-SETMAR protein, a fragment and a peptidomimetic thereof.

In a further embodiment, the αS-SETMAR agonist is a nucleic acid which comprises an expression cassette comprising a polynucleotide encoding αS-SETMAR protein, said polynucleotide being operatively linked to at least one transcriptional regulatory sequence.

As used herein, the term "**αS-SETMAR protein**" refers to any naturally occurring isoform of the αS-SETMAR protein, including the protein of SEQ ID NO: NO: 4, allelic variants thereof, splice variants thereof and orthologous αS-SETMAR protein is highly conserved among higher primates. s. In the context of the invention, the man skilled in the art will readily determine the appropriate αS-SETMAR orthologous protein (or the polynucleotide encoding for such αS-SETMAR protein such as defined by SEQ ID NO: 4) to be used according to the subject to be treated.

In certain embodiments, a **"gene"** refers to a nucleic acid that is transcribed. In certain aspects, the gene comprises a nucleic acid, and/or encodes a polypeptide. In this respect, the term "gene" is used for simplicity to refer to a nucleic acid comprising a nucleotide sequence that is transcribed, the corresponding sequence in RNA bases and the complement thereof. In particular aspects, the transcribed nucleotide sequence comprises at least one functional protein, polypeptide and/or peptide encoding unit. As will be understood by those in the art, this functional term "gene" includes both genomic sequences, RNA (mRNA, Long intergenic non-coding RNAs ...) or cDNA sequences, or smaller engineered nucleic acid segments.

The term **"nucleic acid"** or **"polynucleotide"** will generally refer to at least one molecule or strand of DNA, RNA or a derivative or mimic thereof, comprising at least one nucleobase, such as, for example, a naturally occurring purine or pyrimidine base found in DNA (e.g., adenine "A," guanine "G," thymine "T," and cytosine "C") or RNA (e.g. A, G, uracil "U," and C). The term "nucleic acid" encompasses the terms "oligonucleotide" and "polynucleotide." The term "polynucleotide" refers to at least one molecule of greater than 30 about 100 nucleobases in length. These definitions generally refer to at least one single stranded molecule, but in specific embodiments will also encompass at least one additional strand that is partially, substantially or fully complementary to the at least one single-stranded molecule. Thus, a nucleic acid may encompass at least one double stranded molecule that comprises one or more complementary strand(s) or "complement(s)" of a particular sequence comprising a strand of the molecule.

A nucleic acid may be made by any technique known to one of ordinary skill in the art. Non-limiting examples of synthetic nucleic acid, particularly a synthetic oligonucleotide, include a nucleic acid made by *in vitro* chemical synthesis using phosphotriester, phosphite or phosphoramidite chemistry and solid phase techniques *via* deoxynucleoside H-phosphonate intermediates. A non-limiting example of enzymatically produced nucleic acid include one produced by enzymes in amplification reactions such as PCR^{™} or the synthesis of oligonucleotides. A non-limiting example of a biologically produced nucleic acid includes recombinant nucleic acid production in living cells (see for 10 example, Molecular cloning: a laboratory manual. - 4th ed. / Michael R. Green, Joseph Sambrook. 2012 Cold Spring Harbor, N.Y*.).* A nucleic acid may be purified on polyacrylamide gels, cesium chloride centrifugation gradients, or by any other means known to one of ordinary skill in the art (see for example, Molecular cloning: a laboratory manual. - 4th ed. /Michael R. Green, Joseph Sambrook. 2012 Cold Spring Harbor, N.Y*.).* The nucleic acid molecule may be isolated, which means that it is essentially free of other nucleic acids. Essentially free from other nucleic acids means that the nucleic acid molecule is at least about 90%, typically at least about 95% and, and notably at least about 98% free of other nucleic acids. In one embodiment, the molecule is essentially pure, which means that the molecule is free not only of other nucleic acids, but also of other materials used in the synthesis and isolation of the molecule. Materials used in synthesis include, for example, enzymes. Materials used in isolation include, for example, gels, such as SDS-PAGE. The molecule is at least about 90% free, typically at least about 95% free and, and notably at least about 98% free of other nucleic acids and such other materials.

The term **"variants"** includes protein and nucleic acid variants. Variant proteins may be naturally occurring variants, such as splice variants, alleles and isoforms. Variations in amino acid sequence may be introduced by substitution, deletion or insertion of one or more codons into the nucleic acid sequence encoding the protein that results in a change in the amino acid sequence of the protein. Variant proteins may be a protein having a conservative or non-conservative substitution. Variant proteins may include proteins that have at least about 80% amino acid sequence identity with a polypeptide sequence disclosed herein. A variant protein may have at least about 80%, 81%, 82%, 83%, 84%, 85%, 86%, 87%, 88%, 89%, 90%, 91%, 92%, 93%, 94%, 95%, 96%, 97%, 98%, 99% amino acid sequence identity to a full-length polypeptide sequence or a fragment of a polypeptide sequence as disclosed herein. Amino acid sequence identity is defined as the percentage of amino acid residues in the variant sequence that are identical with the amino acid residues in the reference sequence, after aligning the sequences and introducing gaps, if necessary, to achieve the maximum percent sequence identity, and not considering any conservative substitutions as part of the sequence identity. Sequence identity may be determined over the full length of the variant sequence, the full length of the reference sequence, or both. The percentage of identity for protein sequences may be calculated by performing a pairwise global alignment based on the Needleman-Wunsch alignment algorithm to find the optimum alignment (including gaps) of two sequences along their entire length, for instance using Needle, and using the BLOSUM62 matrix with a gap opening penalty of and a gap extension penalty of 0.5.

Variant nucleic acid sequences may include nucleic acid sequences that have at least about 80% nucleic acid sequence identity with a nucleic acid sequence disclosed herein. A variant nucleic acid sequence may have at least about 80%, 81%, 82%, 83%, 84%, 85%, 86%, 87%, 88%, 89%, 90%, 91%, 92%, 93%, 94%, 95%, 96%, 97%, 98%, 99% nucleic acid sequence identity to a full-length nucleic acid sequence or a fragment of 15 a nucleic acid sequence as disclosed herein. Nucleic acid sequence identity can be calculated by methods well-known to one of skill in the art. The percentage of identity may be calculated by performing a pairwise global alignment based on the Needleman- Wunsch alignment algorithm to find the optimum alignment (including gaps) of two sequences along their entire length, for instance using Needle, and using the DNAFULL matrix with a gap opening penalty of 10 and a gap extension penalty of 0.5.

The term **"fragments"** includes protein and nucleic acid fragments. A protein sequence may be truncated at the N-terminus or C-terminus, or may lack internal residues, for example, when compared with a full length protein. Preferably, said fragments are at least about 10, 20, 30, 40, 50, 60, 70, 80, 90, 100, 110, 120, 139, 149, 150 amino acids in length.

Level of expression of a protein or a polypeptide may be assessed by using immunologic methods such as detection using polyclonal or monoclonal antibodies, chimeric antibody or humanized antibodies. The level of expression of the αS-SETMAR protein is quantified using technologies well known by the art. Suitable immunologic methods include enzyme linked immunoassays (ELISA), sandwich, direct, indirect, or competitive ELISA assays, enzyme linked immunospotassays (ELIspot), radio immunoassays (RIA), flow-cytometry assays (FACS), immunohistochemistry, Western Blot, fluorescence resonance energy transfer (FRET) assays, protein chip assays using for example antibodies, antibody fragments, receptor ligands or other agents binding the αS-SETMAR proteins.

In another embodiment the αS-SETMAR agonist is a nucleic acid.

In another embodiment the αS-SETMAR agonist is a nucleic acid having SEQ ID NO:3.

For example, the αS-SETMAR agonist may be a nucleic acid which comprises a polynucleotide encoding αS-SETMAR protein. Typically, the αS-SETMAR agonist may be an expression cassette comprising said polynucleotide.

In another embodiment, the αS-SETMAR agonist may be a polypeptide such as a dominant activated mutant of αS-SETMAR, a wild-type αS-SETMAR protein, a fragment or a peptidomimetic thereof.

As used herein the term **"protein"** or **"polypeptide"** refers to any chain of amino acids linked by peptide bonds, regardless of length or post-translational modification. Polypeptides include natural proteins, synthetic or recombinant polypeptides and peptides (i.e. polypeptides of less than 50 amino acids) as well as hybrid, post-translationally modified polypeptides, and peptidomimetic.

As used herein the term **"peptidomimetic"** refers to peptide-like structures which have non-amino acid structures substituted but which mimic the chemical structure of the peptide and retain the functional properties of the peptide such as for example, the αS-SETMAR protein.

Peptidomimetics may be designed in order to increase peptide stability, bioavailability, solubility, etc. Typically, the αS-SETMAR agonist is a polypeptide encoded by a nucleic acid. For example, said nucleic acid may be an expression cassette. **"Expression cassette"** according to the invention refers to a linear or circular nucleic acid molecule. This expression cassette also refers to DNA and RNA sequences which are capable of allowing the production of a functional nucleotide sequence in a suitable host cell. Typically, the expression cassette comprises a polynucleotide encoding a mutant of αS-SETMAR such as a dominant activated mutant of αS-SETMAR, a wild-type αS-SETMAR protein, or a fragment thereof, said polynucleotide being operatively linked to at least one transcriptional regulatory sequence. The polynucleotide may comprise the sequence **SEQ ID NO : 4.** Typically, the expression cassette comprises a polynucleotide encoding αS-SETMAR protein, said polynucleotide being operatively linked to at least one transcriptional regulatory sequence for the expression of αS-SETMAR protein in target cells, said at least one transcriptional regulatory sequence being 3'UTR and/or 5'UTR αS-SETMAR sequences.

The expression cassette can also include sequences required for proper translation of the nucleotide sequence of interest. The expression cassette may additionally contain selection marker genes. Typically, the cassette comprises in the 5' -3' direction of transcription, a transcriptional and translation initiation region, a polynucleotide encoding the αS-SETMAR protein, a transcription and translation termination region functional in mammalian cells.

The expression cassette may also include a multiple cloning site.

In addition to the components mentioned above, the expression cassette of the present invention may comprise the components required for homologous recombination. The term **"operatively linked to"** refers to the functional relationship of a nucleic acid with another nucleic acid sequence. Promoters, enhancers, transcriptional and translational stop sites, and other signal sequences are examples of nucleic acid sequences operatively linked to other sequences. For example, operative linkage of DNA to a transcriptional control element refers to the physical and functional relationship between the DNA and promoter such that the transcription of such DNA is initiated from the promoter by an RNA polymerase that specifically recognizes, binds to and transcribes 10 the DNA.

As used herein, the term **"transcriptional regulatory sequence",** "transcription regulatory sequence » or "regulatory sequences" refers to nucleotide sequences influencing the transcription, RNA processing or stability, or translation of the associated (or functionally linked) nucleotide sequence to be transcribed. The transcriptional regulatory sequence may have various localizations with the respect to the nucleotide sequences to be transcribed. The transcriptional regulatory sequence may be located upstream (5' non-coding sequences), within, or downstream (3' non-coding sequences) of the sequence to be transcribed (e.g., polynucleotide encoding αS-SETMAR protein). The transcription regulating nucleotide sequences may be selected from the group consisting of enhancers, promoters, translation leader sequences, introns, 5' -untranslated sequences (5' UTR), 3' -untranslated sequences (3' UTR), and polyadenylation signal sequences. They include natural and synthetic sequences as well as sequences, which may be a combination of synthetic and natural sequences. As is noted above, the term "transcriptional regulatory sequence" is not limited to promoters. However, transcriptional regulatory sequence of the invention may comprise at least one promoter sequence (e.g., a sequence localized upstream of the transcription start of a gene capable to induce transcription of the downstream sequences), and/or at least one 3' UTR and/or one 5' UTR.

In one preferred embodiment the transcription regulating nucleotide sequence of the invention comprises the promoter sequence of the αS-SETMAR gene and/or the native 3'UTR of αS-SETMAR gene and/or native 5'UTR of αS-SETMAR gene. Furthermore, a fragment of the αS-SETMAR 3'UTR and/or of the αS-SETMAR 5'UTR may also be employed.

As used herein, the term **"Promoter"** or **"promoter sequence"** refers to a DNA sequence in a gene, usually upstream (5') to its coding sequence, which controls the transcription of the coding sequence such as the polynucleotide encoding αS-SETMAR protein by providing the recognition for RNA polymerase and other factors required for proper transcription. For example, the promoter may be the αS-SETMAR promoter, a variant or a fragment thereof, preferably, the human αS-SETMAR promoter. Promoters may contain DNA sequences that are involved in the binding of protein factors which control the effectiveness of transcription initiation in response to physiological or developmental conditions. Typically, the αS-SETMAR promoter may contain two GC boxes 5 which are bound by Sp1 and Sp3 factors.

According to the invention, the promoter sequence may also contain enhancer elements. An **"enhancer"** is a DNA sequence which can stimulate promoter activity. It may be an innate element of the promoter or a heterologous element inserted to enhance the level and/or tissue-specificity of a promoter. Typically, the promoter sequence of the invention is a ubiquitous promoter, a tissue-specific promoter or an inducible promoter. "Ubiquitous Promoters" refers to those that direct gene expression in all tissues and at all times. The ubiquitous promoter may be eukaryotic or viral promoters.

In one embodiment, the promoter sequence is eukaryotic promoter selected from the group consisting of the chicken β-actin promoter (CBA), the composite CAG promoter (consisting of the CMV immediate early enhancer and the chicken β-actin promoter) and the human phosphoglycerate kinase 1 (PGK) promoter. According to another embodiment, the promoter sequence is a viral promoter such as the human cytomegalovirus (CMV) promoter.

The term **"Tissue-specific"** promoters as referred to herein are those that direct gene expression almost exclusively in specific tissues, such as CNS (Central Nervous System) specific promoter.

Typically, the promoter sequence is an **"Inducible promoters"** refers to those that direct gene expression in response to an external stimulus, such as light, heat-shock and chemical.

The **"untranslated region"** or "**UTR**" refers to either of the two regions immediately adjacent to the coding sequence on a strand of mature mRNA. When it is found on the 5' side, it is called the 5' UTR (or 5' untranslated region), or if it is found on 30 the 3' side, it is called the 3' UTR (or trailer sequence). As used herein, the term "**3'UTR αS-SETMAR**" refers to the sequence of the 3'UTR of the αS-SETMAR gene, such as for example, the human αS-SETMAR 3'UTR.

As used herein, the term "**5'UTR αS-SETMAR**" refers to the sequence of the 5'UTR of the **αS-SETMAR** gene, such as for example, the human **αS-SETMAR** 5'UTR

In one embodiment, the expression cassette is comprised in an expression vector.

The term **"vector"** refers to a nucleic acid sequence capable of transporting into a cell another nucleic acid to which the vector sequence has been linked. The term **"expression vector"** includes any vector containing a gene construct or an expression cassette in a form suitable for expression by a cell. The "expression vector" may be any recombinant vector capable of expression of a αS-SETMAR protein or fragment thereof.

As used herein, **"delivering",** "gene delivery", "gene transfer", "transducing" can refer to the introduction of an exogenous polynucleotide into a host cell, irrespective of the method used for the introduction. Such methods include a variety of well-known techniques such as vector-mediated gene transfer (e.g., viral infection/transfection, or vanous other protein-based or lipid-based gene delivery complexes) as well as techniques facilitating the delivery of "naked" polynucleotides (e.g., electroporation, "gene gun" delivery and various other techniques used for the introduction of polynucleotides). The introduced polynucleotide may be stably or transiently maintained in the host cell. Stable maintenance typically requires that the introduced polynucleotide either contains an origin of replication compatible with the host cell or integrates into a replicon of the host cell such as an extrachromosomal replicon (e.g., a plasmid) or a nuclear or mitochondrial chromosome.

More particularly, the expression vectors used can be derived from bacterial plasmids, transposons, yeast episomes, from insertion elements, from yeast chromosomal elements, from viruses such as an adeno-associated virus (AAV) vector, a lentivirus vector, a retrovirus vector, a replication competent adenovirus vector, a replication deficient adenovirus vector and a gutless adenovirus vector, a herpes virus vector, baculoviruses, blinked as SV40 virus, the vaccinia virus, fox pox viruses, pseudorabies viruses. AAV and lentivirus vectors have emerged as the vectors of choice for gene transfer to the central nervous system as they mediate efficient long-term gene expression with no apparent toxicity.

AAV, a helper-dependent single-stranded DNA parvovirus, has never been shown to cause disease in humans or animals. The vector is able to durably and efficiently induce gene expression in dividing or terminally differentiated cells. It has been proven to be well tolerated with benign immune response.

The expression cassette can be inserted into the expression vector by methods well known in the art.

The expression vector may include reporter genes. Examples of reporter genes encode luciferase, (green/red) fluorescent protein and variants thereof, like eGFP (enhanced green fluorescent protein), hrGFP (humanized recombinant green fluorescent protein), RFP (red fluorescent protein, like DsRed or DsRed2), CFP (cyan fluorescent protein), BFP (blue fluorescent protein), YFP (yellow fluorescent protein), β-galactosidase or chloramphenicol acetyltransferase, and the like. These sequences are selected depending on the host cell implemented.

According to one embodiment of the invention, the expression vector is a viral vector. The viral vector of the invention may be derived from retroviruses, herpes simplex viruses, adenoviruses or AAVs. According to the present invention, these vectors are particularly advantageous. In one embodiment, the expression vector of the invention is an AAV vector comprising respectively the 5' inverted terminal repeat (ITR5') and 3' inverted terminal repeat (ITR3') sequences of the AAV, at the 5' and 3' ends of said expression cassette.

As used herein, the terms **"AAV vector"** or **"AAV particle"** or **"AAV plasmid"** refer to the nucleic acid derived from any adeno-associated virus vector or any vector derived from an adeno-associated virus. The term **"Terminal inverted repeat sequence"** or "ITR" means the terminal inverted repeat sequences of palindromic 145 base-pairs (bp) flanked at the 5 ' and 3' AAV vector according to the invention. The ITRs sequences are essential for the integration, replication and packaging of the viral vector. AAV ITR's can be modified using standard molecular biology techniques. Accordingly, AAV ITRs used in the vectors of the invention need not have a wild-type nucleotide sequence, and may be altered, e.g., by the insertion, deletion or substitution of nucleotides. Indeed, the ITR5 ' and ITR3 ' are not necessarily identical but are functional. "Functional ITR sequences" means ITR sequences that allow vector replication and packaging. Additionally, AAV ITRs may be derived from any of several AAV serotypes, including but not limited to AAV-1, AAV-2, AAV-3, AAV-4, AAV5, AAV8 or AAV-9.

In another embodiment, the expression cassette is in a viral particle. Typically, the expression cassette inserted into an expression vector which is packaged or encapsidated in a viral particle.

As used herein, the **"viral particle"** refers to the packaged or encapsidated viral vector that is capable of binding to the surface and entering inside the host cells. The techniques for isolating viral particles of this invention from host cellular constituents and eventually from other types of viruses (such as helper viruses) which may be present in the host cell, are known to those of skill in the art, and include, for example, centrifugation and affinity chromatography. Typically, the viral particle may be an AAV particle. The expression "adeno-associated virus" or "AAV" or "AAV particle" means non-enveloped single-stranded DNA belonging to the family Parvoviridae virus and Dependovirus genus. Wild-type AAVs are low integrative viruses but not lytic and non-pathogenic to humans. They infect a wide variety of mitotic and quiescent cells but are dependent on a helper virus for their replication, such as adenovirus or herpes virus. As used herein, the term **"rAAV"** refers to a recombinant AAV-nucleic acid molecule containing some AAV sequences, usually at a minimum the ITRs and some foreign or exogenous (i.e., non-AAV) DNA, such as the αS-SETMAR nucleic acid sequence of the Invention

As used herein, the term **"serotype"** refers to an AAV which is identified by and distinguished from other AAVs based on capsid protein reactivity with defined antisera. The adeno-associated virus serotype, including without limitation, AAV-1, AAV-2, AAV-3, AAV-4, AAV-5, AAV6, AAV7, AAV8, AAV9, AAV10, AAVrh.lO, etc. AAV vectors can have one or more of the AAV wild-type genes deleted in whole or part, e.g. the rep and/or cap genes, but retain functional flanking ITR sequences. Functional ITR sequences are necessary for the rescue, replication and packaging of the AAV virion. Thus, an AAV vector is defined herein to include at least those sequences required in cis for replication and packaging (e. g., functional ITRs) of the virus. ITRs do not need to be the wild-type polynucleotide sequences, and may be altered, e.g, by the insertion, deletion or substitution of nucleotides, so long as the sequences provide for functional rescue, replication and packaging. AAV expression vectors are constructed using known techniques to at least provide as operatively linked components in the direction of transcription, control elements including a transcriptional initiation region, the DNA of interest (i.e. the nucleic acid sequences of the invention) and a transcriptional termination region.

In certain embodiments the viral vectors utilized in the compositions and methods of the invention are recombinant adeno-associated virus (rAAV). The rAAV may be of any serotype, modification, or derivative, known in the art, or any combination thereof (e.g., a population of rAAV that comprises two or more serotypes, e.g., comprising two or more of rAAV2, rAAV8, and rAAV9) known in the art. In some embodiments, the rAAV are rAAVl, rAAV2, rAAV3, rAAV4, rAAV5, rAAV6, rAAV7, rAAV8, rAAV9, rAAVIO, rAAV-11, rAAV-12, rAAV-13, rAAV- 14, rAAV- 15, rAAV-16, rAAV.rh8, rAAV.rhlO, rAAV.rh20, rAAV.rh39, rAAV.Rh74, rAAV.RHM4-l, AAV.hu37, rAAV.Anc80, rAAV.Anc80L65, rAAV.7m8, rAAV.PHP.B, rAAV2.5, rAAV2tYF, rAAV3B, rAAV.LK03, rAAV.HSCl, rAAV.HSC2, rAAV.HSC3, rAAV.HSC4, rAAV.HSC5, rAAV.HSC6, rAAV.HSC7, rAAV.HSC8, rAAV.HSC9, rAAV.HSCIO , rAAV.HSCl 1, rAAV.HSC12, rAAV.HSC13, rAAV.HSC14, rAAV.HSC15, or rAAV.HSCl 6, or other rAAV, or combinations of two or more thereof.

In some embodiments, the rAAV used in the compositions and methods of the invention comprise a capsid protein from an AAV capsid serotype selected from AAV1, AAV2, AAV3, AAV4, AAVS, AAV6, AAV7, AAV8, AAV9, AAV10, AAV-11, AAV- 12, AAV- 13, AAV-14, AAV-15, AAV- 16, AAV.rh8, AAV.rhlO, AAV.rh20, AAV.rh39, AAV.Rh74, AAV.RHM4-1, AAV.hu37, AAV.Anc80, AAV.Anc80L65, AAV.7m8, AAV.PHP.B, AAV2.5, AAV2tYF, AAV3B, AAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, or AAV.HSC16, or a derivative, modification, or pseudotype thereof. In some embodiments, the rAAV comprise a capsid protein at least 80% or more identical, e.g., 85%, 85%, 87%, 88%, 89%, 90%, 91%, 92%, 93%, 94%, 95%, 96%, 97%, 98%, 99%, 99.5%, etc., i.e. up to 100% identical, to e.g., vpl, vp2 and/or vp3 sequence of an AAV capsid serotype selected from AAV1, AAV2, AAV3, AAV4, AAV5, AAV6, AAV7, AAV8, AAV9, AAV10, AAV-11, AAV- 12, AAV- 13, AAV-14, AAV-15, AAV- 16, AAV.rh8, AAV.rhlO, AAV.rh20, AAV.rh39, AAV.Rh74, AAV.RHM4-1, AAV.hu37, AAV.Anc80, AAV.Anc80L65, AAV.7m8, AAV.PHP.B, AAV2.5, AAV2tYF, AAV3B, AAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, or AAV.HSC16.

In certain embodiments, the AAV that is used in the methods described herein is Anc80 or Anc80L65, as described in Zinn et al., 2015: 1056-1068, which is incorporated by reference in its entirety. In certain embodiments, the AAV that is used in the methods described herein comprises one of the following amino acid insertions: LGETTRP (SEQ ID NO: 14) or LALGETTRP (SEQ ID NO: 15), as described in United States Patent Nos. 9,193,956; 9458517; and 9,587,282 and US patent application publication no. 2016/0376323, each of which is incorporated herein by reference in its entirety. In certain embodiments, the AAV that is used in the methods described herein is AAV.7m8, as described in United States Patent Nos. 9,193,956; 9,458,517; and 9,587,282 and US patent application publication no. 2016/0376323, each of which is incorporated herein by reference in its entirety. In certain embodiments, the AAV that is used in the methods described herein is any AAV disclosed in United States Patent No. 9,585,971, such as AAV-PHP.B. In certain embodiments, the AAV that is used in the methods described herein is any AAV disclosed in United States Patent No. 9,840,719 and WO 2015/013313, such as AAV.Rh74 and RHM4-1, each of which is incorporated herein by reference in its entirety. In certain embodiments, the AAV that is used in the methods described herein is any AAV disclosed in WO 2014/172669, such as AAV rh.74, which is incorporated herein by reference in its entirety. In certain embodiments, the AAV that is used in the methods described herein is AAV2/5, as described in Georgiadis et al., 2016, Gene Therapy 23: 857-862 and Georgiadis et al, 2018, Gene Therapy 25: 450, each of which is incorporated by reference in its entirety. In certain embodiments, the AAV that is used in the methods described herein is any AAV disclosed in WO 2017/070491, such as AAV2tYF, which is incorporated herein by reference in its entirety. In certain embodiments, the AAV that is used in the methods described herein is AAVLK03 or AAV3B, as described in Puzzo et al, 2017, Sci. Transl. Med. 29(9): 418, which is incorporated by reference in its entirety. In certain embodiments, the AAV that is used in the methods described herein is any AAV disclosed in US Pat Nos. 8,628,966; US 8,927,514; US 9,923,120 and WO 2016/049230, such as HSC1, HSC2, HSC3, HSC4, HSC5, HSC6, HSC7, HSC8, HSC9, HSC10, HSC11, HSC12, HSC13, HSC14, HSC15, or HSC16, each of which is incorporated by reference in its entirety.

In certain embodiments, the AAV that is used in the methods described herein is an AAV disclosed in any of the following patents and patent applications, each of which is incorporated herein by reference in its entirety: United States Patent Nos. 7,282,199; 7,906,111; 8,524,446; 8,999,678; 8,628,966; 8,927,514; 8,734,809; US 9,284,357; 9,409,953; 9,169,299; 9,193,956; 9458517; and 9,587,282; US patent application publication nos. 2015/0374803; 2015/0126588; 2017/0067908; 2013/0224836; 2016/0215024; 2017/0051257; and International Patent Application Nos. PCT/US2015/034799; PCT/EP2015/053335. In some embodiments, the rAAV have a capsid protein at least 80% or more identical, e.g., 85%, 85%, 87%, 88%, 89%, 90%, 91%, 92%, 93%, 94%, 95%, 96%, 97%, 98%, 99%, 99.5%, etc., i.e. up to 100% identical, to the vpl, vp2 and/or vp3 sequence of an AAV capsid disclosed in any of the following patents and patent applications, each of which is incorporated herein by reference in its entirety: United States Patent Nos. 7,282,199; 7,906,111; 8,524,446; 8,999,678; 8,628,966; 8,927,514; 8,734,809; US 9,284,357; 9,409,953; 9,169,299; 9,193,956; 9458517; and 9,587,282; US patent application publication nos. 2015/0374803; 2015/0126588; 2017/0067908; 2013/0224836; 2016/0215024; 2017/0051257; and International Patent Application Nos. PCT/US2015/034799; PCT/EP2015/053335.

In some embodiments, the rAAV have a capsid protein disclosed in Inti. Appl. Publ. No. WO 2003/052051 (see, e.g., SEQ ID NO: 2), WO 2005/033321 (see, e.g., SEQ ID NOs: 123 and 88), WO 03/042397 (see, e.g., SEQ ID NOs: 2, 81, 85, and 97), WO 2006/068888 (see, e.g., SEQ ID NOs: 1 and 3-6), WO 2006/110689, (see, e.g., SEQ ID NOs: 5-38) WO2009/104964 (see, e.g., SEQ ID NOs: 1-5, 7, 9, 20, 22, 24 and 31), WO 2010/127097 (see, e.g., SEQ ID NOs: 5-38), and WO 2015/191508 (see, e.g., SEQ ID NOs: 80-294), and U.S. Appl. Publ. No. 20150023924 (see, e.g., SEQ ID NOs: 1, 5-10), the contents of each of which is herein incorporated by reference in its entirety. In some embodiments, the rAAV have a capsid protein at least 80% or more identical, e.g., 85%, 85%, 87%, 88%, 89%, 90%, 91%, 92%, 93%, 94%, 95%, 96%, 97%, 98%, 99%, 99.5%, etc., i.e. up to 100% identical, to the vpl, vp2 and/or vp3 sequence of an AAV capsid disclosed in Inti. Appl. Publ. No. WO 2003/052051 (see, e.g., SEQ ID NO: 2), WO 2005/033321 (see, e.g., SEQ ID NOs: 123 and 88), WO 03/042397 (see, e.g., SEQ ID NOs: 2, 81, 85, and 97), WO 2006/068888 (see, e.g., SEQ ID NOs: 1 and 3-6), WO 2006/110689 (see, e.g., SEQ ID NOs: 5-38) WO2009/104964 (see, e.g., SEQ ID NOs: 1-5, 7, 9, 20, 22, 24 and 31), WO 2010/127097 (see, e.g., SEQ ID NOs: 5-38), and WO 2015/191508 (see, e.g., SEQ ID NOs: 80-294), and U.S. Appl. Publ. No. 20150023924 (see, e.g., SEQ ID NOs: 1, 5-10).

Nucleic acid sequences of AAV based viral vectors and methods of making recombinant AAV and AAV capsids are taught, for example, in United States Patent Nos. 7,282,199; 7,906,111; 8,524,446; 8,999,678; 8,628,966; 8,927,514; 8,734,809; US 9,284,357; 9,409,953; 9,169,299; 9,193,956; 9458517; and 9,587,282; US patent application publication nos. 2015/0374803; 2015/0126588; 2017/0067908; 2013/0224836; 2016/0215024; 2017/0051257;

International Patent Application Nos. PCT/US2015/034799; PCT/EP2015/053335; WO 2003/052051, WO 2005/033321, WO 03/042397, WO 2006/068888, WO 2006/110689, WO2009/104964, WO 2010/127097, and WO 2015/191508, and U.S. Appl. Publ. No. 20150023924.

In additional embodiments, the rAAV comprise a pseudotyped rAAV. In some embodiments, the pseudotyped rAAV are rAAV2/8 or rAAV2/9 pseudotyped rAAV. Methods for producing and using pseudotyped rAAV are known in the art (see, e.g., Duan et al., J. Virol., 75:7662-7671 (2001); Halbert et al., J. Virol., 74:1524-1532 (2000); Zolotukhin et al., Methods 28:158-167 (2002); and Auricchio et al., Hum. Molec. Genet. 10:3075-3081, (2001).

In additional embodiments, the rAAV comprise a capsid containing a capsid protein chimeric of two or more AAV capsid serotypes. In some embodiments, the capsid protein is a chimeric of 2 or more AAV capsid proteins from AAV serotypes selected from AAV1, AAV2, AAV3, AAV4, AAV5, AAV6, AAV7, AAV8, AAV9, AAV10, AAV-11, AAV- 12, AAV-13, AAV- 14, AAV-15, AAV-16, AAV.rh8, AAV.rhlO, AAV.rh20, AAV.rh39, AAV.Rh74, AAV.RHM4-1, AAV.hu37, AAV.Anc80, AAV.Anc80L65, AAV.7m8, AAV.PHP.B, AAV2.5, AAV2tYF, AAV3B, AAV.LK03, AAV.HSC1, AAV.HSC2, AAV.HSC3, AAV.HSC4, AAV.HSC5, AAV.HSC6, AAV.HSC7, AAV.HSC8, AAV.HSC9, AAV.HSC10, AAV.HSC11, AAV.HSC12, AAV.HSC13, AAV.HSC14, AAV.HSC15, or AAV.HSC16.

In certain embodiments, a single-stranded AAV (ssAAV) can be used. In certain embodiments, a self-complementary vector, e.g., scAAV, can be used (see, e.g., Wu, 2007, Human Gene Therapy, 18(2): 171-82, McCarty et al, 2001, Gene Therapy, Vol. 8, Number 16, Pages 1248-1254; and U.S. Patent Nos. 6,596,535; 7,125,717; and 7,456,683, each of which is incorporated herein by reference in its entirety).

In certain embodiments, the recombinant AAV vector used for delivering the transgene have a tropism for cells in the DRG. Such vectors can include non-replicating "rAAV", particularly those bearing an AAV8 or AAVrhlO capsid are preferred. In certain embodiments, the viral vectors provided herein are AAV9 or AAVrhlO based viral vectors. In certain embodiments, the AAV8 or AAVrhlO based viral vectors provided herein retain tropism for DRG. AAV variant capsids can be used, including but not limited to those described by Wilson in US Patent No. 7,906,111 which is incorporated by reference herein in its entirety, with AAV/hu.31 and AAV/hu.32 being particularly preferred; as well as AAV variant capsids described by Chatterjee in US Patent No. 8,628,966, US Patent No. 8,927,514 and Smith et al., 2014, Mol Ther 22: 1625-1634, each of which is incorporated by reference herein in its entirety.

In some embodiment, the present invention relates to a recombinant adeno-associated virus (rAAV) comprising (i) an expression cassette containing a transgene under the control of regulatory elements and flanked by ITRs, and (ii) an AAV capsid, wherein the transgene encodes αS-SETMAR mRNA and activates expression of αS-SETMAR in a cell.

Provided in particular embodiments are AAV vectors comprising an artificial genome comprising (i) an expression cassette containing the transgene under the control of regulatory elements and flanked by ITRs; and (ii) a viral capsid that has the amino acid sequence of the AAV capsid protein or is at least 95%, 96%, 97%, 98%, 99% or 99.9% identical to the amino acid sequence of the AAV capsid protein while retaining the biological function of the AAV capsid.

Provided in particular embodiments are AAVrhlO vectors comprising an artificial genome comprising (i) an expression cassette containing the transgene under the control of regulatory elements and flanked by ITRs; and (ii) a viral capsid that has the amino acid sequence of the AAVrhlO capsid protein or is at least 95%, 96%, 97%, 98%, 99% or 99.9% identical to the amino acid sequence of the AAVrhlO capsid protein while retaining the biological function of the AAVrhlOcapsid. In certain embodiments, the encoded AAVrhlO capsid has the sequence of SEQ ID NO: 81 set forth in U.S. Patent No. 9,790,427 which is incorporated by reference herein in its entirety, with 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acid substitutions and retaining the biological function of the AAVrhlO capsid.

In another embodiment, lentiviral vectors are employed.

As used herein, the term **"lentiviral vector"** refers to a vector derived from (i.e.,sharing nucleotides sequences unique to) a lentivirus. The term "lentiviral vector" also refers to a modified lentivirus having a modified proviral RNA genome which comprises a NGB polynucleotide sequence. According to the invention, the lentiviral vectors derivative from the human immunodeficiency virus (HIV). Lentiviruses are complex retroviruses, which, in addition to the common retroviral genes gag, pol, and env, contain other genes with regulatory or structural function. The higher complexity enables the virus to modulate its life cycle, as in the course of latent infection. Some examples of lentivirus include the Human Immunodeficiency Viruses (HIV 1, HIV 2) and the Simian Immunodeficiency Virus (SIV). Lentiviral vectors have been generated by multiply attenuating the HIV virulence genes, for example, the genes env, vif, vpr, vpu and nef are deleted making the vector biologically safe. Lentiviral vectors are known in the art, see, e.g.. U.S. Pat. Nos. 6,013,516 and 5,994,136, both of which are incorporated herein by reference. In general, the vectors are plasmid-based or virus-based, and are configured to carry the essential sequences for incorporating foreign nucleic acid, for selection and for transfer of the nucleic acid into a host cell. The gag, pol and env genes of the vectors of interest also are known in the art. Thus, the relevant genes are cloned into the selected vector and then used to transform the target cell of interest. Recombinant lentivirus capable of infecting a non-dividing cell wherein a suitable host cell is transfected with two or more vectors carrying the packaging functions, namely gag, pol and env, as well as rev and tat is described in U.S. Pat. No. 5,994,136, incorporated herein by reference. This describes a first vector that can provide a nucleic acid encoding a viral gag and a pol gene and another vector that can provide a nucleic acid encoding a viral env to produce a packaging cell. Introducing a vector providing a heterologous gene into that packaging cell yields a producer cell which releases infectious viral particles carrying the foreign gene of interest. The env preferably is an amphotropic envelope protein which allows transduction of cells of human and other species. Typically, the nucleic acid molecule or the vector of the present invention include "control sequences", which refers collectively to promoter sequences, polyadenylation signals, transcription termination sequences, upstream regulatory domains, origins of replication, internal ribosome entry sites ("IRES"), enhancers, and the like, which collectively provide for the replication, transcription and translation of a coding sequence in a recipient cell. Not all of these control sequences need always be present so long as the selected coding sequence is capable of being replicated, transcribed and translated in an appropriate host cell.

As used herein, a **"host cell"** refers to any cell that harbors, or is capable of harboring, the expression cassette of the invention, the expression vector of the invention, or the αS-SETMAR nucleic acid sequence or any cell that express, or is capable of expressing αS-SETMAR protein. The term **"host cell"** also refers to a cell that has been transformed, or is capable of transformation, by an exogenous nucleic acid molecule such as the isolated polynucleotide of the invention. Host cells containing the transformed polynucleotide are referred to as "transgenic" host cells. Said host cells may be used to obtain organisms which are not human or may be obtained from said organisms. The host cell may be a prokaryotic cell (bacteria or cyanobacteria) or a eukaryotic cell (e.g. fungi, algae, yeast, plant, mammalian or insect cells). Typically, a mammal cell may be a rodent (mouse, rat), a feline, a canine or a primate cell. A mammal cell may be selected from the group of cell lines, tissue, somatic cells, neuron or neuronal derived cells, retinal cells and glial cells.

In another embodiment, the αS-SETMAR agonist according to the invention is a mRNA.

As used herein, the term **"mRNA"** refers to a single-stranded molecule of RNA that corresponds to the genetic sequence of a gene, and is read by a ribosome in the process of synthesizing a protein.

In the context of the invention, the mRNA corresponds to the genetic sequence of αS-SETMAR.

In a particular embodiment, the invention relates to a method for treating glioblastoma in a subject in need thereof, comprising administering to the subject a therapeutically effective amount of a composition comprising mRNA encoding αS-SETMAR, wherein the mRNA is delivered via a nanoparticle-based delivery system.

In a particular embodiment, the method according to the invention, wherein the nanoparticle-based delivery system comprises: lipid nanoparticles encapsulating the mRNA, optionally, a targeting ligand that directs the nanoparticles to glioblastoma cells.

Typically, in the context of the invention, mRNA encoding for αS-SETMAR is delivered by ultrasound. This method leverages a phenomenon called transient cavitation, where ultrasound waves create microbubbles that temporarily disrupt cell membranes, allowing mRNA to enter the cells more efficiently.

In another embodiment, the αS-SETMAR agonist according to the invention is an antisens oligonucleotides Antisense oligonucleotides (ASOs). Anti-sense oligonucleotides, including anti-sense RNA molecules and anti-sense DNA molecules, act directly to block the translation of the targeted mRNA. Such ASO is able to target the sequence of αS-SETMAR which is deleterious for the αS-SETMAR activity.

As used herein, the term **"oligonucleotide"** refers to an oligomer of the nucleotides defined above. The term "oligonucleotide" refers to a nucleic acid sequence, 3'-5' or 5'-3' oriented, which may be single- or double-stranded. The oligonucleotide used in the context of the invention may in particular be DNA or RNA. The term also includes "oligonucleotide analog" which refers to an oligonucleotide having (i) a modified backbone structure, e.g. , a backbone other than the standard phosphodiester linkage found in natural oligo- and polynucleotides, and (ii) optionally, modified sugar moieties, e.g., morpholino moieties rather than ribose or deoxyribose moieties. Oligonucleotide analogs support bases capable of hydrogen bonding by Watson-Crick base pairing to standard polynucleotide bases, where the analog backbone presents the bases in a manner to permit such hydrogen bonding in a sequence-specific fashion between the oligonucleotide analog molecule and bases in a standard polynucleotide {e.g., single-stranded RNA or single-stranded DNA). Particularly, analogs are those having a substantially uncharged, phosphorus containing backbone. A substantially uncharged, phosphorus containing backbone in an oligonucleotide analog is one in which a majority of the subunit linkages, e.g., between 50-100%, typically at least 60% to 100% or 75% or 80% of its linkages, are uncharged, and contain a single phosphorous atom.

The term **"oligonucleotide"** also refers to an oligonucleotide sequence that is inverted relative to its normal orientation for transcription and so correspond to a RNA or DNA sequence that is complementary to a target gene mRNA molecule expressed within the host cell (e.g., it can hybridize to the target gene mRNA molecule through Watson-Crick base pairing).

An antisense strand can be constructed in a number of different ways, provided that it is capable of interfering with the expression of a target gene. For example, the antisense strand can be constructed by reverse-complementing the coding region (or a portion thereof) of the target gene relative to its normal orientation for transcription to allow the transcription of its complement, (e.g., RNAs encoded by the antisense and sense gene may be complementary). In some embodiments, the oligonucleotide need not have the same intron or exon pattern as the target gene, and noncoding segments of the target gene may be equally effective in achieving antisense suppression of target gene expression as coding segments such as antisense oligonucleotide (ASO). In some embodiments, the oligonucleotide has the same exon pattern as the target gene such as antisense oligonucleotide (ASO).

In other embodiments, the oligonucleotide is targeted to a translation initiation site (AUG codon), sequences in the coding region (*e.g.* one or more exons), 5'-untranslated region or 3'-untranslated region of an mRNA. The aim is to interfere with functions of the messenger RNA include all vital functions including translocation of the RNA to the site for protein translation, actual translation of protein from the RNA, splicing or maturation of the RNA and possibly even independent catalytic activity which may be engaged in by the RNA. The overall effect of such interference with the RNA function is to cause interference with protein expression.

In some embodiments, the oligonucleotide of the present invention is further modified, particularly chemically modified, in order to increase the stability and/or therapeutic efficiency in vivo. The one skilled in the art can easily provide some modifications that will improve the efficacy of the oligonucleotide such as stabilizing modifications (C. Frank Bennett and Eric E. Swayze, RNA Targeting Therapeutics: Molecular Mechanisms of Antisense Oligonucleotides as a Therapeutic PlatformAnnu. Rev. Pharmacol. Toxicol. 2010.50:259-293; Juliano RL. The delivery of therapeutic oligonucleotides. Nucleic Acids Res. 2016 Aug 19;44(14):6518-48). In particular, the oligonucleotide used in the context of the invention may comprise modified nucleotides. Chemical modifications may occur at three different sites: (i) at phosphate groups, (ii) on the sugar moiety, and/or (iii) on the entire backbone structure of the oligonucleotide. Typically, chemical modifications include backbone modifications, heterocycle modifications, sugar modifications, and conjugation strategies.

For example the oligonucleotide is be selected from the group consisting of oligodeoxyribonucleotides, oligoribonucleotides, small regulatory RNAs (sRNAs), U7- or U1-mediated ASOs or conjugate products thereof such as peptide-conjugated or nanoparticle-complexed ASOs, chemically modified oligonucleotide by backbone modifications such as morpholinos, phosphorodiamidate morpholino oligomers (Phosphorodiamidate morpholinos, PMO), peptide nucleic acid (PNA), phosphorothioate (PS) oligonucleotides, stereochemically pure phosphorothioate (PS) oligonucleotides, phosphoramidates modified oligonucleotides, thiophosphoramidate-modified oligonucleotides, and methylphosphonate modified oligonucleotides; chemically modified oligonucleotide by heterocycle modifications such as bicycle modified oligonucleotides, Bicyclic Nucleic Acid (BNA), tricycle modified oligonucleotides, tricyclo-DNA-antisense oligonucleotides (ASOs), nucleobase modifications such as 5-methyl substitution on pyrimidine nucleobases, 5-substituted pyrimidine analogues, 2-Thio-thymine modified oligonucleotides, and purine modified oligonucleotides; chemically modified oligonucleotide by sugar modifications such as Locked Nucleic Acid (LNA) oligonucleotides, 2',4'-Methyleneoxy Bridged Nucleic Acid (BNA), ethylene-bridged nucleic acid (ENA), constrained ethyl (cEt) oligonucleotides, 2'-Modified RNA, 2'- and 4'-modified oligonucleotides such as 2'-O-Me RNA (2'-OMe), 2'-O-Methoxyethyl RNA (MOE), 2'-Fluoro RNA (FRNA), and 4'-Thio-Modified DNA and RNA; chemically modified oligonucleotide by conjugation strategies such as N-acetyl galactosamine (GalNAc) oligonucleotide conjugates such as 5'-GalNAc and 3'-GalNAc ASO conjugates, lipid oligonucleotide conjugates (LASO), cell penetrating peptides (CPP) oligonucleotide conjugates, targeted oligonucleotide conjugates, antibody-oligonucleotide conjugates, polymer-oligonucleotide conjugate such as with PEGylation and targeting ligand; and chemical modifications and conjugation strategies described for example in Bennett and Swayze, 2010 (RNA targeting therapeutics: molecular mechanisms of antisense oligonucleotides as a therapeutic platform. Annu Rev Pharmacol Toxicol. 2010;50:259-93); Wan and Seth, 2016 (The Medicinal Chemistry of Therapeutic Oligonucleotides. J Med Chem. 2016 Nov 10;59(21):9645-9667); Juliano, 2016 (The delivery of therapeutic oligonucleotides. Nucleic Acids Res. 2016 Aug 19;44(14):6518-48); Lundin et al., 2015 (Oligonucleotide Therapies: The Past and the Present. Hum Gene Ther. 2015 Aug;26(8):475-85); and Prakash, 2011 (An overview of sugar-modified oligonucleotides for antisense therapeutics. Chem Biodivers. 2011 Sep;8(9):1616-41). Indeed, for use in vivo, the oligonucleotide may be stabilized. A "stabilized" oligonucleotide refers to an oligonucleotide that is relatively resistant to in vivo degradation (e.g. via an exo- or endo-nuclease). Stabilization can be a function of length or secondary structure. In particular, oligonucleotide stabilization can be accomplished via phosphate backbone modifications, phosphodiester modifications, phosphorothioate (PS) backbone modifications, combinations of phosphodiester and phosphorothioate modifications, thiophosphoramidate modifications, 2' modifications (2'-O-Me, 2'-O-(2-methoxyethyl) (MOE) modifications and 2'-fluoro modifications), methylphosphonate, methylphosphorothioate, phosphorodithioate, p-ethoxy, and combinations thereof.

In a particular embodiment, the antisense oligonucleotide is lipid-conjugated, known as LASO. In some embodiments, the antisense oligonucleotide of the present invention is modified by substitution at the 3' or the 5' end by a moiety comprising at least three saturated or unsaturated, particularly saturated, linear or branched, particularly linear, hydrocarbon chains comprising from 2 to 30 carbon atoms, particularly from 5 to 20 carbon atoms, more particularly from 10 to 18 carbon atoms as described in WO2014/195432.

In some embodiments, the antisense oligonucleotide of the present invention is modified by substitution at the 3' or the 5' end by a moiety comprising at least one ketal functional group, wherein the ketal carbon of said ketal functional group bears two saturated or unsaturated, particularly saturated, linear or branched, particularly linear, hydrocarbon chains comprising from 1 to 22 carbon atoms, particularly from 6 to 20 carbon atoms, in particular 10 to 19 carbon atoms, and even more particularly from 12 to 18 carbon atoms as described in WO2014/195430.

For example, the oligonucleotide may be employed as phosphorothioate derivatives (replacement of a non-bridging phosphoryl oxygen atom with a sulfur atom), which have increased resistance to nuclease digestion. 2'-methoxyethyl (MOE) modification (such as the modified backbone commercialized by IONIS Pharmaceuticals) is also effective. Additionally or alternatively, the oligonucleotide of the present invention may comprise completely, partially or in combination, modified nucleotides which are derivatives with substitutions at the 2' position of the sugar, in particular with the following chemical modifications: O-methyl group (2'-O-Me) substitution, 2-methoxyethyl group (2'-O-MOE) substitution, fluoro group (2'-fluoro) substitution, chloro group (2'-Cl) substitution, bromo group (2'-Br) substitution, cyanide group (2'-CN) substitution, trifluoromethyl group (2'-CF3) substitution, OCF3 group (2'-OCF3) substitution, OCN group (2'-OCN) substitution, O-alkyl group (2'-O-alkyl) substitution, S-alkyl group (2'-S-alkyl) substitution, N-alkyl group (2'-N-akyl) substitution, O-alkenyl group (2'-O-alkenyl) substitution, S-alkenyl group (2'-S-alkenyl) substitution, N-alkenyl group (2'-N-alkenyl) substitution, SOCH3 group (2'-SOCH3) substitution, SO2CH3 group (2'-SO2CH3) substitution, ONO2 group (2'-ONO2) substitution, NO2 group (2'-NO2) substitution, N3 group (2'-N3) substitution and/or NH2 group (2'-NH2) substitution. Additionally or alternatively, the oligonucleotide of the present invention may comprise completely or partially modified nucleotides wherein the ribose moiety is used to produce locked nucleic acid (LNA), in which a covalent bridge is formed between the 2' oxygen and the 4' carbon of the ribose, fixing it in the 3'-endo configuration. These molecules are extremely stable in biological medium, able to activate RNase H such as when LNA are located to extremities (Gapmer) and form tight hybrids with complementary RNA and DNA.

In some embodiments, the oligonucleotide used in the context of the invention comprises modified nucleotides selected from the group consisting of LNA, 2'-OMe analogs, 2'-O-Met, 2'-O-(2-methoxyethyl) (MOE) oligomers, 2'-phosphorothioate analogs, 2'-fluoro analogs, 2'-Cl analogs, 2'-Br analogs, 2'-CN analogs, 2'-CF3 analogs, 2'-OCF3 analogs, 2'-OCN analogs, 2'-O-alkyl analogs, 2'-S-alkyl analogs, 2'-N-alkyl analogs, 2'-O-alkenyl analogs, 2'-S-alkenyl analogs, 2'-N-alkenyl analogs, 2'-SOCH3 analogs, 2'-SO2CH3 analogs, 2'-ONO2 analogs, 2'-NO2 analogs, 2'-N3 analogs, 2'-NH2 analogs, tricyclo (tc)-DNAs, U7 short nuclear (sn) RNAs, tricyclo-DNA-oligoantisense molecules and combinations thereof (U.S. Provisional Patent Application Serial No. 61/212,384 For: Tricyclo-DNA Antisense Oligonucleotides, Compositions and Methods for the Treatment of Disease, filed April 10, 2009, the complete contents of which is hereby incorporated by reference).

In a particular embodiment, the oligonucleotide according to the invention is a LNA oligonucleotide. As used herein, the term "LNA" (Locked Nucleic Acid) (or "LNA oligonucleotide") refers to an oligonucleotide containing one or more bicyclic, tricyclic or polycyclic nucleoside analogues also referred to as LNA nucleotides and LNA analogue nucleotides. LNA oligonucleotides, LNA nucleotides and LNA analogue nucleotides are generally described in International Publication No. WO 99/14226 and subsequent applications; International Publication Nos. WO 00/56746, WO 00/56748, WO 00/66604, WO 01/25248, WO 02/28875, WO 02/094250, WO 03/006475; U.S. Patent Nos. 6,043,060, 6268490, 6770748, 6639051, and U.S. Publication Nos. 2002/0125241, 2003/0105309, 2003/0125241, 2002/0147332, 2004/0244840 and 2005/0203042, all of which are incorporated herein by reference. LNA oligonucleotides and LNA analogue oligonucleotides are commercially available from, for example, Proligo LLC, 6200 Lookout Road, Boulder, CO 80301 USA.

Typically, such ASO is used in the exon skipping strategy.

As used herein, the term **"exon skipping"** masks specific exons during the splicing process of pre-mRNA, resulting in the exclusion (skipping) of these exons from the mature mRNA. This can restore the reading frame of the mRNA and produce a functional, albeit truncated, protein.

In another aspect, the invention relates to i)an agonist of αS-SETMAR and ii) a classical treatment for use by simultaneous, separate or sequential administration in the prevention and/or treatment of cancer and/or metastatic cancer in a subject in need thereof.

In a particular embodiment, the invention relates to i) an agonist of αS-SETMAR and ii) a classical treatment for use by simultaneous, separate or sequential administration in the prevention and/or treatment of glioblastoma and/or metastatic glioblastoma in a subject in need thereof.

As used herein, the terms **"combined treatment", "combined therapy"** or **"therapy combination"** refer to a treatment that uses more than one medication.

As used herein the terms **"administering"** or **"administration"** refer to the act of injecting or otherwise physically delivering a substance as it exists outside the body (e.g., an agonist of αS-SETMAR) into the subject, such as by, oral, intravenous, intramuscular, enteral, subcutaneous, parenteral, systemic, local, spinal, nasal, topical or epidermal administration (e.g., by injection or infusion). When a disease, or a symptom thereof, is being treated, administration of the substance typically occurs after the onset of the disease or symptoms thereof. When a disease or symptoms thereof, are being prevented, administration of the substance typically occurs before the onset of the disease or symptoms thereof. In a particular embodiment, the administration is performed by a patches, a paste, an ointment, a suspension, a solution or a cream, a gel or a spray. In a particular embodiment, the administration is performed by a cream.

In a particular embodiment, the administration of the αS-SETMAR is performed by an intrathecal, subcutaneous, topical or intravenous administration.

As used herein, the term **"administration simultaneously"** refers to administration of 2 active ingredients by the same route and at the same time or at substantially the same time. The term **"administration separately"** refers to an administration of 2 active ingredients at the same time or at substantially the same time by different routes. The term **"administration sequentially"** refers to an administration of 2 active ingredients at different times, the administration route being identical or different.

Typically, the agonist of αS-SETMAR is selected from the group consisting of but not limited to: polypeptide, nucleic acid, mRNA or ASO. Such agonists are described above.

Typically, the classical treatment refers to immunotherapy such as anti-immune checkpoint therapy, chemotherapy, radiotherapy or surgery.

As used herein, the terms **"treating"** or **"treatment"** refer to both prophylactic or preventive treatment as well as curative or disease modifying treatment, including treatment of subject at risk of contracting the disease or suspected to have contracted the disease as well as subject who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a subject during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a subject during treatment of an illness, e.g., to keep the subject in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

In another aspect, the invention relates to a pharmaceutical composition which comprises the αS-SETMAR agonist according to the invention.

In a particular embodiment, the invention relates to a pharmaceutical composition comprising αS-SETMAR agonist for treating glioblastoma.

In a particular embodiment, the invention relates to a pharmaceutical composition which comprises the αS-SETMAR agonist according to the invention and a classical treatment as a combined preparation for treating glioblastoma.

The αS-SETMAR agonist as described above may be combined with pharmaceutically acceptable excipients, and optionally sustained-release matrices, such as biodegradable polymers, to form pharmaceutical compositions. "Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. The pharmaceutical compositions of the present invention for *per os* (oral), sublingual, subcutaneous, intramuscular, intravenous, transdermal, local or rectal administration, the active principle, alone or in combination with another active principle, can be administered in a unit administration form, as a mixture with conventional pharmaceutical supports, to subjects, such as animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

In a particular embodiment, the pharmaceutical composition according to the invention is administered by an intrathecal, subcutaneous, topical or intravenous administration.

Typically, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The polypeptide (or nucleic acid encoding thereof) can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. Sterile injectable solutions are prepared by incorporating the active polypeptides in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation may be vacuum-drying and freeze-drying techniques which yield a powder of the active ingredient plus any additional desired ingredient from a previously sterile-filtered solution thereof. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

In a particular embodiment, the present invention provides a topical formulation comprising antisense oligonucleotides. For example, and not by way of limitation, the present invention provides a topical formulation comprising antisense oligonucleotides. Dosage forms for the topical or transdermal administration of the inhibitors of the present invention include, but are not limited to, powders, sprays, ointments, pastes, creams, lotions, gels, solutions, patches and inhalants. In certain non-limiting embodiments, a topical formulation comprises antisense oligonucleotides comprised in micelles, liposomes, or non-lipid based microspheres. In certain non-limiting embodiments, such a topical formulation may comprise a permeability enhancing agent such as but not limited to dimethyl sulfoxide, hydrocarbons (for example, alkanes and alkenes), alcohols (for example, glycols and glycerols), acids (for example, fatty acids), amines, amides, esters (for example, isopropyl myristate), surfactants (for example, anionic, cationic, or non- ionic surfactants), terpenes, and lipids (for example, phospholipids).

The invention will be further illustrated by the following figures and examples. However, these examples and figures should not be interpreted in any way as limiting the scope of the present invention.

### FIGURES:

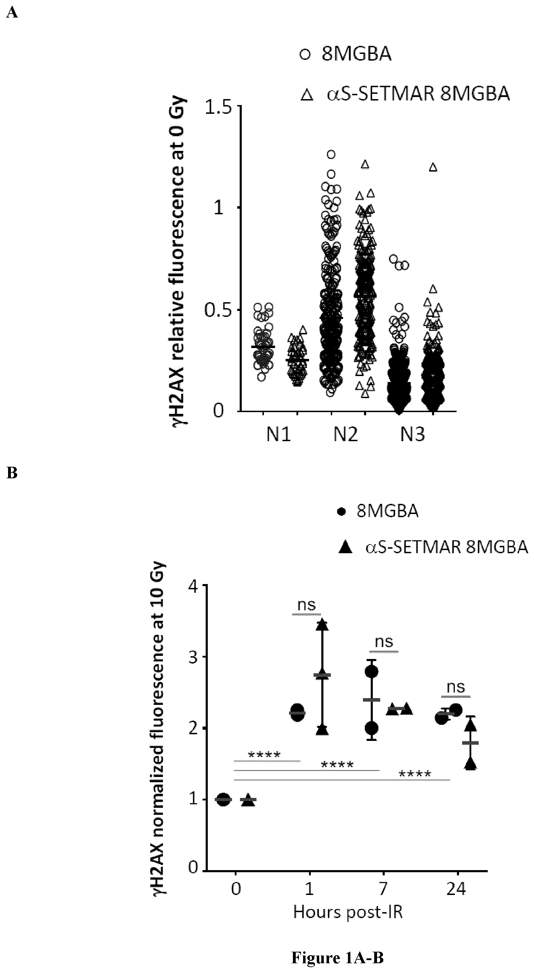

**Fig 1****. αS-SETMAR does not modify DSB repair.** A. Quantification of γ-H2AX foci fluorescence intensity at 0 Gy in 8MGBA (green dots) and αS-SETMAR-8MGBA (orange dots) cells, using ImageJ software. Scatter plots of all points are shown, for each assay (N). Means are shown as black bars. Within each assay (N), statistical analyses reveal no differences between both cell lines (Wilcoxon matched-pairs signed rank test). **B.** Quantification of γ - H2AX foci fluorescence intensity at 1, 7 and 24 h post-irradiation (10 Gy) in 8MGBA (green dots) and S-SETMAR-8MGBA (orange dots) cells, using ImageJ software. For each point post-irradiation (time and cell line), the mean fluorescence intensity is calculated within each assay (N1, N2 and N3) and normalized against the mean value at 0 Gy of the same assay. For each time post-irradiation, statistical analyses reveal no differences between both cell lines (Wilcoxon matched-pairs signed rank test). **** indicates a p value < 0.0001 (Wilcoxon matched-pairs signed rank test), for both cell line between T0 and the corresponding post-irradiation time.

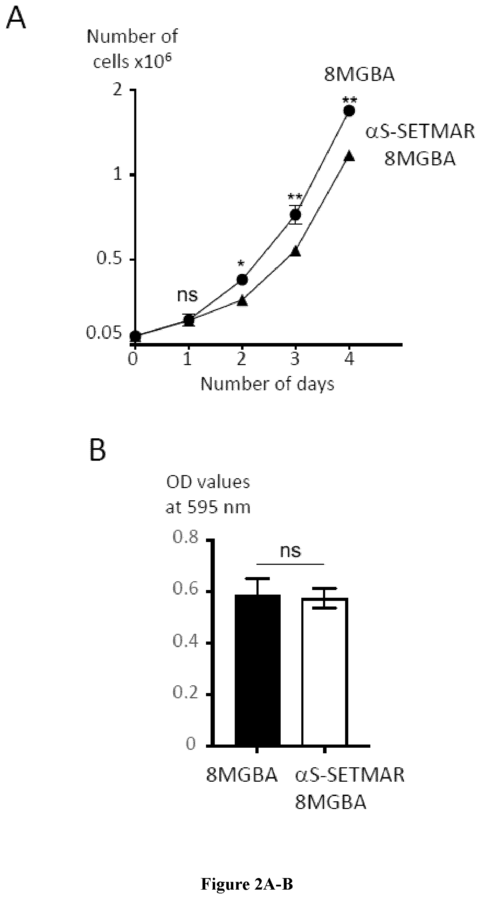

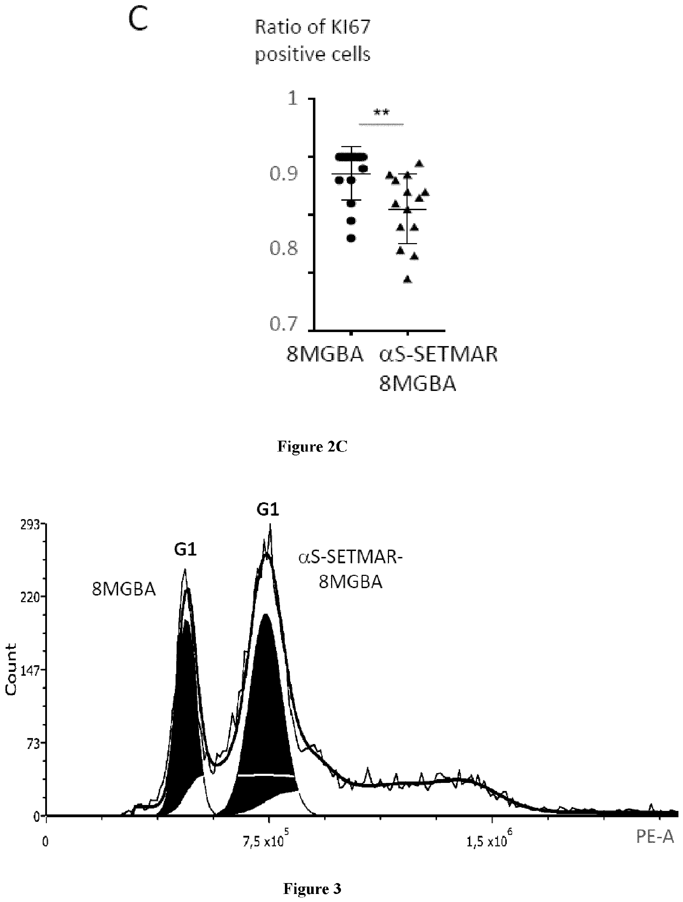

**Fig 2****. αS-SETMAR modulates cell behavior.** A. Growth curves of 8MGBA (green) and αS-SETMAR-8MGBA (orange) cells grown in standard conditions. From the second day, the number of αS-SETMAR-8MGBA cells was significantly lower than that of 8MGBA (N=6; Mann-Whitney at J2, p=0.015; J3, p=0.0087; J4, p=0.0022). B. MTT assay of 8MGBA (green) and αS-SETMAR-8MGBA (orange) cells. Histograms show the means of OD values at 595 nm, with no differences between cell lines (N=3, Mann-Whitney test). C. Proliferation of 8MGBA (green) and αS-SETMAR-8MGBA (orange) revealed by KI67 IHC staining. Scatter dot plots show the rate of Ki67 positive cells in both cell lines (n=15, p=0.0014 in Mann-Whitney test).
**Fig 3****. αS-SETMAR triggers aneuploidy.** FACS analysis of a mixture of αS-SETMAR-8MGBA and 8MGBA control cells, with the same 8MGBA and αS-SETMAR-8MGBA.

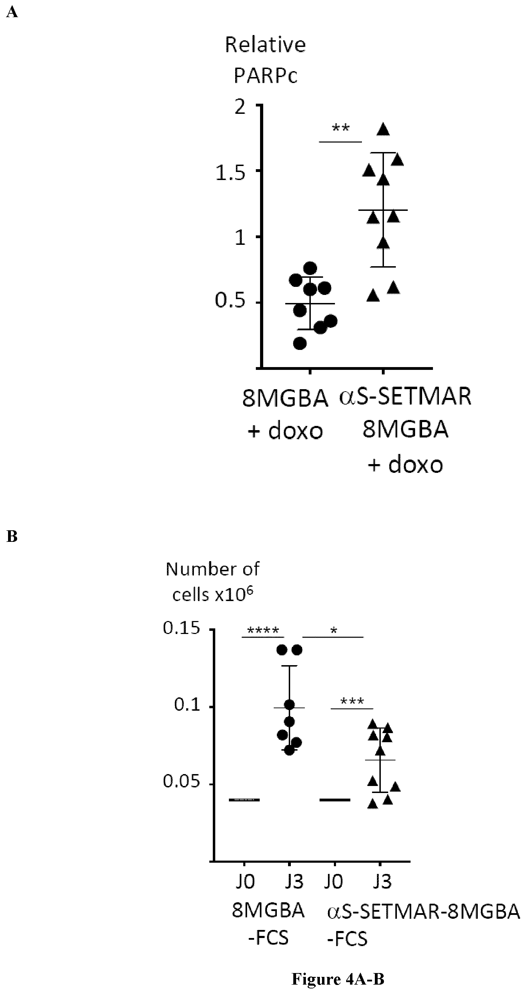

**Fig 4****. αS-SETMAR and cell death. A.** Apoptosis assay of 8MGBA and αS-SETMAR-8MGBA cells. PARPc signals were detected by Western blot for cells treated (+) or not (-) with 1µM doxorubicin (Doxo). Scatter dot plots show the normalized PARPc signal for both cell lines treated with Doxorubicin (N=8, p=0.0025 in Mann-Whitney test). **B.** Serum starvation. Cells were platted without FCS for 3 days. Scatter dot plots show the number of living cells for both serum-starved cell lines (N=3, n=7 or 9). 8MGBA: J0 versus J3, p<0.00001 and αS-SETMAR-8MGBA: J0 versus J3, p=0.0003 in Mann-Whitney test). Serum starvation has more effect on αS-SETMAR-8MGBA than on 8MGBA cells (p=0.028 in Mann-Whitney test).

### EXAMPLE:

### Material & Methods

### Biological resources

8MGBA (human GB, ACC-432 DSMZ) cells were cultured in Minimum Essential Medium (MEM, ThermoFisher) supplemented with 10 % Fetal Calf Serum (FCS, ThermoFisher) and maintained in 5 % CO2 at 37°C. For stable transfection, the αS-SETMAR sequence was cloned downstream of the pEF1 promoter of the pEF1-V5-HisA plasmid (Invitrogen, V92020) giving pEF1-V5-αS-SETMAR (plasmid map in S1). Plasmid was transfected in 8MGBA using Fugene^{®} HD (Promega), per manufacturer's instruction. After 24h, cells were transferred to 100 mm dishes and the medium was supplemented with 1200 µg/mL of neomycin (G418, Invitrogen). After 2 weeks of selection, single foci were picked and grown in a 24-well plate. The stable expression of αS-SETMAR was confirmed by RT-q-PCR and Western blot. The resulting αS-SETMAR-8MGBA cell lines are routinely maintained in MEM supplemented with 10 % FCS and 800 µg/ µL neomycin. 8MGBA cells containing an empty pEF1-V5-HisA plasmid were similarly prepared and used as control. In the main text, we used the name "αS-SETMAR" to appoint the protein over-expressed in our assays, and "S-SETMAR" to appoint the short variant describe in literature and for which the presence/absence of the α-peptide is unknown.

### Reagents

Immunocytochemistry (IHC) for double strand breaks (DSB) detection was performed using an anti-γ-H2AX pS139 antibody (ab11174, Abcam, 1/1 000) for the primary antibody, and a donkey anti-rabbit secondary antibody coupled to fluorophore (Alexafluor^{™}, ThermoFisher, A-10040, 1/500) for the second antibody. IHC for cell proliferation was performed using an anti-Ki67 antibody (ab 16667, Abcam, 1/500) for the primary antibody, and a goat anti-mouse secondary antibody coupled to fluorophore (Alexafluor^{™}, ThermoFisher, A-11005, 1/200) for the second antibody. For Western blots (WB), we used the following primary antibodies: anti-SETMAR (Ab129455, Abcam, 1/2 500), anti-actin-HRP (A3854, Sigma-Aldrich, 1/100 000), anti-α-peptide (custom designed by Covalab, 1/1 000; described in [3]), anti-PARPc (mAb 5625, Cell signaling, 1/1 000), and an anti-rabbit IgG, HRP conjugate as secondary antibody (Promega, W401B, 1/2 500).

### Proteins extraction and Western blot analysis

Cells were lysed with Radio Immuno-Precipitation Assay (RIPA) buffer (1 % NP-40, 0.1 % SDS, 0.5 % Na-DOC, 20 mM HEPES buffer, pH 7.5, 150 mM NaCl, and 1X HALT inhibitor protease cocktail (Pierce)) for 30 min at 4°C. Cellular debris were removed by centrifugation (13 000 g for 10 min at 4°C) and the supernatant (crude protein extracts) recovered in a fresh tube. Protein concentrations were assayed using the BCA Protein Assay Kit (DC protein Assay, Bio-Rad) per manufacturer's instructions, using the microplate procedure. For WB assays, 20 µg of protein extracts were separated on 4-20 % polyacrylamide gels and transferred to nitrocellulose membranes. The immunoblots were revealed using the ECL purity kit (Bio-Rad). Membranes were imaged with a Chemidoc Touch equipment (Bio-Rad) and signals were quantified with the Image J software [17].

### Irradiation

100 000 8MGBA or αS-SETMAR-8MGBA cells were seeded per 6-well plate on coverslips for upcoming IHC and irradiated 24 h later in a single dose. RX-650 irradiator (Faxitron X-Ray LLC) was used to deliver 1.7 Gy/min. IC experiments were performed 1 h, 7 h and 24 h after irradiation as indicated. Non-irradiated cells were used as control.

### Proliferation tests

80 000 8MGBA or αS-SETMAR-8MGBA cells were seeded per 4-well chamber slides (Nunc Lab-Tek) for upcoming IHC and incubated in standard conditions for 24 h.

### Immunocytochemistry (IHC)

Cells were fixed with 4 % paraformaldehyde (ThermoFisher) and 4 % sucrose (Sigma-Aldrich) for 20 min at room temperature (RT), permeabilized in PBS solution containing 0.2 % Triton100 (Sigma-Aldrich) for 15 min and blocked in 5 % Bovine Serum Albumin (BSA, Sigma-Aldrich) for 20 min. Hybridizations were realized in PBS containing 1 % BSA and the primary antibody for 2 h. After washing (in PBS), cells were incubated in PBS containing 1 % BSA and the secondary antibody coupled to fluorophore for 1 h. Cells were then mounted with the DAPI ProLong^{™} Diamond Antifade Mountant solution (ThermoFisher). After drying, immunofluorescence (IF) was imaged with a Nikon Eclipse Ti microscope and captured with a Nikon digital SightDS-Ri1 camera. Signals from antibodies were quantified with ImageJ software [17], and normalized against DAPI signals.

### Growth rates and MTT tests

50 000 8MGBA and αS-SETMAR-8MGBA cells were plated in triplicate in 12-wells plates. At 24 h, 48 h, 72 h cells were harvested, 10 µL of each well were diluted (1/2) in Trypan Blue and counted with Kovaslides. The number of dead and live cells was assessed, the mean of each replicate was calculated, and the total number of cells was determined. The assay was repeated twice. The remaining cells were used to performed the MTT assay: 40 000 cells were collected and incubated for 1 h at 37°C in MEM+FCS and MTT (Invitrogen, M6494) at 0.5 mg/mL and centrifugated 5 min at 1100 rpm. The pellet was resuspended in DMSO (the volume was adjusted to obtain DO<1), transferred in 96-wells plates and the absorbance at 595 nm was measured.

### Karyotyping and aCGH array studies

Karyotyping G banding was performed using standard methods on metaphase spreads from growth phase of 8MGBA and αS-SETMAR-8MGBA cell lines. Genomic DNA was extracted using Nucleo Spin Tissue kit (Macherey Nagel). Array comparative genomic hybridization (aCGH) experiments were performed using Agilent Human Genome CGH 180 K oligonucleotide arrays with 8MGBA DNA as control (Agilent, Santa Clara, CA; www.agilent.com). Custom array has a probe every 13 kb. The arrays were analyzed with the Agilent scanner and the Feature Extraction software (v.9.1.3). Graphical overview was obtained using the CGH analytics software (v.3.5.14).

### Cell tracking by timelapse

50 000 8MGBA cells and 75 000 αS-SETMAR-8MGBA cells were plated in 6-wells plates for 24 h prior to the timelapse in 3 mL of appropriate culture media. The timelapse was realized with a microscope equipped with a CO2 chamber (Evos M5000, Invitrogen). For 8MGBA cells pictures were taken every 5 min for 67.25 h and every 10 min for S-SETMAR-8MGBA cells for 91.30 h. Throughout the experiment, the chamber was supplied with 5 % CO2, without hygrometry for 8MGBA cells and 85 % for αS-SETMAR-8MGBA cells. Timelapse was analyzed as previously described [18]. When possible, grand-daughters cells were included in the analysis. Cell cycle duration was determined by the time between two anaphases.

### Cell cycle analysis by Fluorescence-Activated Cell Sorting (FACS)

Asynchronous 8MGBA and αS-SETMAR-8MGBA cells were cultured for 72 h in T25 flasks then harvested by scratching and centrifugated 5 min at 1000 g at RT. The cells were then treated with the Cell Cycle Analysis Kit (Sigma-Aldrich, MAK344). The protocol was adapted, centrifugations were performed at 1000 g and 250 µL of Staining solution was used. Cells were stored at -20°C until marking. In order to allow tune comparisons, pool sample was made by pooling 8MGBA and of αS-SETMAR-8MGBA cells (v/v) after fixation in ethanol. This sample was processed as describe above. Cells were sorted with BD AccuriTM C6 Plus Flow Cytometer. For each condition, 40 000 events were measured and analyzed. Single cells in cycle were selected using FCS Express. Cell cycle phases were automatically defined by the software. The statistical fitting model with the lowest p-value was used for the control (8MGBA). The selected fitted model was then applied to all conditions. For the pool sample, after selection, single cells in cycle were analyzed with the MultiCycle function. The fitted model was defined by the software. Percentages of cells in the different cell cycle phases was assessed and the mean of each replicate was calculated.

### RNA-seq analysis

RNA were extracted with Direct-Zol RNA miniprep kit (Zymo Research). The RNA quantity was assessed with the Nanodrop 2000 Spectrophotometer (Thermo Fischer Scientific) and the quality of RNA was checked with the Agilent 2100 Bioanalyzer, using the RNA 6000 Nano kit (Agilent Technologies, Les Ulis, France), according to the manufacturers' recommendations. The samples exhibiting an RNA Integrity umber (RIN) between 8 and 10 (three biological replicates per condition from three independent experiments) were used for RNA-seq experiment. This work has benefited from the facilities and expertise of Platform Genomics Paris Centre (IBENS). RNA-seq libraries were performed using TruSeq Stranded mRNA kit (Illumina). Indexed samples were sequenced on Nextseq Illumina 500 to obtain single end reads. Low quality sequences were improved with Trimmomatic (Galaxy Version 0.38.0). Reads were mapped on the human reference genome GRCH38.p11 with HISAT2 (Galaxy Version 2.2.1+galaxy0). Expression level of transcripts was quantified with htseq-count (Galaxy Version 0.9.1) and differential expression was performed with DESEQ2 (Galaxy Version 2.11.40.8). Differentially expressed genes with a p-value adjusted <0,05 and a |Log2FC| >1 were retained for functional analysis. For data analysis, gene lists were recovered using the NCBI (https://www.ncbi.nlm.nih.gov/gene) and the GO (https://geneontology.org/) datasets to upload Homo sapiens genes involved in various cellular processes (cell cycle; G1, S, G2, M phases; G1/S, S/G2, G2/M, metaphasis checkpoints, spindle assembly, chromosome segregation). Gene lists are shown in supplemental data (S1 Table).

### Apoptosis assays

106 8MGBA and αS-SETMAR-8MGBA cells were cultured in T25 flasks for 24 h and then treated (or not, for control) with 1 µM of Doxorubicin (Sigma, D1515-10MG) in 5 mL of fresh medium, for 48 h. The treated cells and respective controls were collected, and the cell pellets lysed in RIPA lysis buffer to extract proteins and measure the appearance of cleaved-PARP (PARPc) as apoptosis landmarks, by WB. Each assay was repeated three times.

### Statistical analyses

The statistical analyses were performed using GraphPad Prism 8 as described in the main text.

### Results

Here, we used the name "αS-SETMAR" to appoint the protein over-expressed in our assays, and "S-SETMAR" to appoint the short variant describe in literature and for which the presence/absence of the α-peptide is unknown. Since it greatly increases αS-SETMAR half-life when compared to S-SETMAR, its presence is therefore a factor influencing the relative quantity of S-SETMAR within the assays. In order to study the role of αS-SETMAR, we have stably modified the 8MGBA cell line to create a line called αS-SETMAR-8MGBA, which constitutively expressed αS-SETMAR in amounts greater than FL-SETMAR (S2). Several clones were recovered and the one having the strongest αS-SETMAR expression (clone 1 in S2) was used for further work.

### S-SETMAR and DNA repair

As mentioned before, the efficiency of S-SETMAR in NHEJ has been already assayed in vitro, using a fusion protein (MBP-S-SETMAR) [9]. As a result, S-SETMAR was found to poorly sustained NHEJ as compared to FL-SETMAR. To verify whether these findings correctly reflect what happens in a cellular context, X-rays irradiations (IR) were previously tested using three single doses (2, 5 and 10 Gy) to determine the one to use. For both cell lines, a decrease in cell viability (87 % for 8MGBA and 83 % for αS-SETMAR-8MGBA cells) was only seen for the 10 Gy dose at 24 h. In addition, this dose induced DNA damage visible by IF γH2AX labelling but did not induce cell death even after 6 days post-IR.

IR were thus performed at 10 Gy on 8MGBA cell lines overexpressing or not αS-SETMAR. γ H2AX foci were detected by IF at 0, 1, 7 and 24 h post-IR, and the extent of DNA repair determined by the decrease of foci intensity [19](**data not shown**). We first verified that the overexpression of αS-SETMAR does not modify the cellular background of double strand breaks (DSB). Three independent assays were performed (N1, N2, N3). For each assay and each cell line, a large variability in the relative fluorescence intensity (y-H2AX/DAPI) of foci was observed, reflecting the variability of each test. Indeed, the mean of each assay revealed no significant difference between the two cell lines (**Fig 1A**). We assumed that αS-SETMAR does not modify the cellular background of DSB. We then analyzed the kinetics of DSB repair for both cell lines. To circumvent the bias due to experimental internal variability previously mentioned, the mean of γ -H2AX relative fluorescence detected at 0 Gy for each assay was normalized to 1 (as the reference). The mean of γ -H2AX relative fluorescence detected at each time after IR was expressed relatively to its own reference. This allowed to give γ -H2AX normalized fluorescence at 10 Gy (**Fig 1B**). We showed that the number of DSB increased by a factor of 2 to 3 after 1 to 7 h (p < 0.0001) for both cell lines, with no significant difference between αS-SETMAR 8MGBA and 8MGBA. 24 h post IR, the level of DSB was still twice that of T0 (p < 0.0001) for both cell lines, again with no significant difference between them. In conclusion, αS-SETMAR does not significantly modify the overall DSB repair. This finding is in agreement with its poor efficiency in triggering NHEJ repair previously shown [9].

### S-SETMAR cellular and molecular effects

In introducing this work, we explained that relationships between FL-SETMAR amount and cell proliferation are ambiguous and probably depend on the S-SETMAR/FL-SETMAR ratio. Considering the SETMAR network and the possible presence of both variants within cells, we assumed that (1) the relative level of both variants may have an impact on cell growth, and (2) each variant may have a different effect. We thus decided to analyze the growth of 8MGBA cells under two conditions: control cells in which FL-SETMAR is predominant (FL>S), and recombinant αS-SETMAR-8MGBA cells, in which αS-SETMAR is very strongly predominant (S>>FL).

As a preliminary step, we checked whether aS/FL-SETMAR heterodimers could assemble in cells co-expressing both variants, a hypothesis that has never been formally demonstrated. Our results (**data not shown**) indicated that such heterodimers may exist, but seemed rare and/or not very stable, actually less stable than both homodimers. The impact of these heterodimers has therefore been neglected in the rest of our study.

Next, we checked for αS-SETMAR overexpression effects in cells proliferation. After two days of culture, we observed a small but significant drop in the number of cells for the αS-SETMAR-8MGBA cell line. This difference increased with time (**Fig 2A**), suggesting that αS-SETMAR has slowed down cells proliferation, either directly or not. To check out the underlying mechanisms, we verified at first if αS-SETMAR decreased 8MGBA viability and/or proliferation. MTT tests revealed no differences between both cell lines viability (**Fig 2B**) whereas Ki67 labelling revealed a **small but significant (p=0.0014) decrease** of αS-SETMAR-8MGBA cells proliferation when compared to 8MGBA cells (**Fig 2C**), in agreement with the slight but significant effect seen in **Fig 2A****.**

As these results were the opposite of the impact of the over-expression of FL-SETMAR [5-7], the transcriptomic impact of αS-SETMAR was carried out by comparing RNA-seq data from 8MGBA *versus* aS-SETMAR-8MGBA cells. Differential expression data indicated that αS-SETMAR overexpression modified the expression of about 2 500 genes (with |LogFC|>1), for a total of 1 494 up-regulated and 1 077 down-regulated genes (**data not shown**). Gene ontology analysis revealed an enrichment in three main groups related to extracellular matrix/cell junctions (307 genes), synapses/axons organization (210 genes) and development/growth regulation (184 genes) (**data not shown**).

We then tested the possibility that the transcriptional control exerted by αS-SETMAR takes place following its binding to a *Hsmarl* TIR. For this purpose, we intersected the gene list given by Tellier *et al* [13] that contained genes with *Hsmarl* TIR retaining at least 80% of the length and identity of the ancestral one, with our list of αS-SETMAR deregulated genes. We found that 2.4 % (61) up-regulated genes contained a *Hsmarl* TIR, compared to 4 % (103) down-regulated ones **(data not shown**). In addition, we confronted the 2 571 αS-SETMAR DEG to those for which an efficient DNA binding has been proven by ChIP-seq analysis. By combining the four studies already published about SETMAR [10,13,15,16], we got a list of 214 genes, 18 of which being also deregulated by αS-SETMAR **(data not shown**). These genes were either transcription factors (5 genes), involved in cell cycle (4 genes) or neurogenesis regulation (6 genes). Among them, 12 (8 down- and 4 up-DEG) contained an *Hsmarl* TIR. Taken together, we identified 170 genes (164 with a TIR and 6 without) for which a direct transcriptional effect can be proposed through the binding of αS-SETMAR homodimers in place of FL-SETMAR homodimers. However, these 170 genes represented only 6.6 % of all DEG, suggesting other regulatory mechanisms, *via* indirect transcriptional effects or alternative pathways, such as the trapping of FL-SETMAR associated proteins, or αS-SETMAR unknown specific effects.

We then assessed whether some genes can be deregulated following the binding of αS-SETMAR to their sequence. The only study [13] that provided crossed data between ChIP and RNA-seq allowed to appoint FL-SETMAR target genes for which DNA binding was correlated to transcription levels modification. Five of them were also deregulated by αS-SETMAR **(data not shown**). Four were deregulated in the same way: *ABTB1, PKHD1* and *SYCP2* (up regulated) and *BDKBB1* (down regulated) and the fifth in the opposite way: *CACNA1A* (down regulated in our data and up regulated in Tellier's data). Interestingly, of the five genes, *CACNA1A* was the only one to be brain-specific, which may explain the divergence in regulation between the two studies, our data having been obtained in CNS (Central Nervous System) cells, whereas those of Tellier in U2OS cells (human osteosarcoma). At this stage of the study, data collected are too disparate to develop a model for transcription regulation by αS-SETMAR especially if the FL-SETMAR activity has to be taken into account.

We then took advantage of the two previous studies that were done in human colorectal cancer cells. In each study, a different SETMAR variant was analyzed by ChIP-seq: recombinant FL-SETMAR in U2OS cells [13] and endogenous S-SETMAR in HT29 cells, which was unique in having this lonely isoform of SETMAR [10]. The available data indicated that 615 genes were targeted by FL-SETMAR (in U2OS cells) and 3 238 by S-SETMAR (in HT29 cells). These values did not necessarily mean that there were more target genes for S-SETMAR than for FL-SETMAR (although this cannot be ruled out), but rather reflected the difference between both experiments, in particular the use of different antibodies, and the low level of recombinant FL-SETMAR expression (as specified by the authors). We identified (with Venn diagram) 359 genes that were bound by both proteins, while 256 were only bound by FL-SETMAR and 2 878 only by S-SETMAR (**data not shown**). These results suggest that some genes were possibly bound by any isoform of the SETMAR proteins, while others seem to be preferentially bound by one or the other. The most amazing point revealed by gene ontology was that target genes of either S-SETMAR or both proteins were mainly involved in CNS development (**data not shown**), even though the cells used for the experiment are not CNS cells. In contrast, no specific enrichment was found for the genes targeted by FL-SETMAR, perhaps due to the low number of genes. Altogether, these observations confirm the involvement of SETMAR in the human embryo brain development, already proposed [4], while suggesting that this role is played by S-SETMAR.

### S-SETMAR slows down the cell cycle

To explain the antiproliferative effects of αS-SETMAR in αS-SETMAR-8MGBA cells, one hypothesis was that they took longer to complete a full cycle, i.e. that their cycle was slowed down compared to that of 8MGBA cells. To verify this point, we carried out time-lapse assays covering a period of time assumed to be necessary to complete at least 2 to 3 full cycles, i.e. 96 hours (**data not shown**). We observed that 8MGBA cells need 27 hours to complete a full cycle (measured between the anaphase of a mother cell and that of its daughter cells), while αS-SETMAR-8MGBA needed 37 hours to complete the same process (**data not shown**). This observation is sufficient to explain why αS-SETMAR-8MGBA cells growth was lower (**Fig 1**) but raises the question of whether a particular phase of the cycle was affected or not. To answer this, FACS analyses have been done (**data not shown**) and revealed no differences between 8MGBA and αS-SETMAR-8MGBA cells. In both cases, the relative amount of cells in G1, S and G2-M phases were about 62-63 %, 29-32 % and 6-7 % respectively, in agreement with previous published data [20].

In an attempt to elucidate the mechanisms that lead to variations in cycle length we looked for deregulated genes involved in the cell cycle phases or checkpoints. Genes involved in cell cycle were selected by combining NCBI (https://www.ncbi.nlm.nih.gov/gene) and GO (http://geneontology.org) datasets. Only the common ones (1 509) were retained for further analysis (**data not shown**). Among these, 60 were up-regulated by αS-SETMAR and 73 down-regulated when compared with our RNA-seq data **(data not shown**). We then investigated these genes distribution in the different phases of the cell cycle and the main checkpoints. Similarly to what was made for the whole cell cycle, gene lists from each phase and/or checkpoint were recovered (**data not shown**) and compared to that of deregulated genes. Results (**data not shown**) revealed that deregulated genes mainly concerned two phases, G1 (12 deregulated genes) and S/replication (11 and 22 DEG respectively), but also the spindle assembly and chromosome segregation processes (33 and 7 DEG respectively). Together, three phases displayed 50 deregulated genes and the checkpoints 48 ones. Within these 98 genes, some were found at more than one stage (phases or checkpoints). By removing duplicates, we found 66 different genes spread across the whole cycle. As we did not identify a specific stage affected by the over-expression of αS-SETMAR **(data not shown),** we looked for a set of genes common to several stages of the cell cycle.

Overall, 17 genes drew attention **(data not shown),** because they were described as being key regulators of the whole cell cycle and identified here in two steps or more **(data not shown**). The four most deregulated genes were related to the interphase.

ANXA1 (known as an effector of glucocorticoid-mediated responses and a regulator of the inflammatory process) was involved in multiple cancer processes, including cell proliferation. In GB, ANXA1 was shown as being overexpressed and negatively associated with poor survival. A recent study [21] reported that its downregulation suppressed GB cell proliferation, in line with our findings. Among the 17 genes retained here, ANXA1 was the most down-regulated (LogFC = -3.3), accounting for the antiproliferative effect related to S-SETMAR.

Hyaluronan, the major component of the extracellular matrix, was thought to be involved in cell proliferation, migration and differentiation and thus may play a role in promoting tumor progression. GB cells have been shown to overproduce hyaluronan, that in turn may constitute a halo around the cells, inducing dendritic cell death and generating an immune-protective barrier for the tumor [22]. In our study HYAL1 (coding for a lysosomal hyaluronidase that intracellularly degraded hyaluronan) is overexpressed in αS-SETMAR-8MGBA cells (LogFC = 1.67). It is tempting to propose that HYAL1 overexpression could prevent hyaluronan overproduction and therefore limited its related proliferative effects.

In cancer, PLK2 (a gene from the polo family of serine/threonine protein kinases) is generally considered as a tumor suppressor, whereas its role in GB is more ambiguous. Recent findings [23] underline that the low expression of PLK2 in GB is due to DNA hypermethylation and predicts favorable prognosis. From the point of view of its mechanism of action, PLK2 is essential for mitotic centriole replication, and its loss leads to cell cycle disorders. In our data, PLK2 was down-regulated by αS-SETMAR overexpression (LogFC = 1.8), in line with the protective effect of S-SETMAR.

The fourth, SUSD2, involved in neuritic outgrowth and excitatory synapse numbers, was found as being significantly down regulated in cancers, and to function as a tumor suppressor. Upon ligand binding, this membrane protein induced G1 cell cycle arrest through inhibition of the cyclin D/CDK6 complex [25]. Overexpression of SUSD2 in αS-SETMAR-8MGBA cells (LogFC = 1.46) was also in line with the protective effect of S-SETMAR.

To finish, the fifth was linked to the whole cycle. RGCC is known to regulate cell cycle progression in many cell types. In the human brain, its expression levels were related to the fate of neural stem cells (NSC) during cortical development between neural [26] and glial lineages [27]. In addition, RGCC KO disrupted the centrosome and spindle organization of NSC during mitosis, and impaired the expression of essential centrosomal proteins. In gliomas, RGCC has also been found located at the centrosome [28]. RGCC was shown as being down regulated in GB [29], but overexpressed in many cancers. Finally, the hypothesis that RGCC could function either as a tumor suppressor or promoter depending on cellular context [30] made it difficult to predict its role in 8MGBA cells. However, the strong down-regulation of RGCC in αS-SETMAR-8MGBA cells (LogFC = -2.48) may impair mitosis through spindle and/or centrosome mis-organization.

In fact, the deregulation of these five genes could be sufficient to explain the cell cycle slowing in αS-SETMAR-8MGBA cells and were in line with the protective effect of S-SETMAR in GB. Nevertheless, none of them was found to be bound by SETMAR in the ChIP-seq list, nor to contain a TIR in their sequence, ruling out a possible direct effect. Instead, indirect effects, through interaction with partner-proteins or disrupting interaction with FL-SETMAR partner-proteins must be assumed. Interestingly, three of them were recognized as players in centriole, centrosome or spindle functions: ANXA1 allows mitotic spindle orientation during mammalian epithelial morphogenesis [31], PLK2 is essential for mitotic centriole replication [32] and the protein RGCC has been found at the centrosome [28], as is believed for SETMAR [13].

A sixth gene, TTN, merited further examination, since it had a TIR in its sequence, opening the possibility of αS-SETMAR direct binding. TITINS (TTN) are giant elastic proteins, the largest known in human, for which a nuclear isoform has been described. Nuclear TTN associates with chromosomes and is essential for mitotic chromosome condensation and segregation. Studies in drosophila implicate TTN in the organization, stability, elasticity and mechanics of both the nuclear envelope and chromosomes during interphase, and mitosis [33]. The down regulation of TTN in αS-SETMAR-8MGBA cells (LogFC = -1.39) was in line with the slowdown in the cell cycle linked to αS-SETMAR.

### S-SETMAR triggers genome chaos

FACS analyses have revealed an intriguing detail, which cannot be due to the longer cycle for αS-SETMAR-8MGBA cells. We were surprised to find a shift to the right on the x-axis of the peaks of each phase, as if αS-SETMAR-8MGBA cells contained 1.5 to 2 times more DNA than 8MGBA (Fig 3). This was confirmed with a αS-SETMAR-8MGBA independent clone (5), which showed the same shift in the DNA amount (S5). To find out more this unexpected observation, two sets of analyses were conducted.

Firstly, the karyotype of both cell lines was determined. 8MGBA cells are known as hyper-diploid cells with about 15% aneuploidy, containing 47-52 chromosomes (DSMZ, https://www.dsmz.de/). 8MGBA control cells conformed to this description, since they contained 49 chromosomes (data **not** shown). In contrast, αS-SETMAR-8MGBA cells contained 77 to 80 chromosomes (data **not** shown), thus displaying an increased aneuploidy correlated to their greater amount of DNA (x1.6). It is worth noting that karyotypes of αS-SETMAR-8MGBA cells were difficult to establish as chromosomes were largely broken and modified. However, comparison of both αS-SETMAR-8MGBA clones (1 versus 5) revealed that the aneuploidy did not concern the same chromosomes, suggesting either that an original event with variable consequences had occurred for each newly established clone (during the production of stable cell lines) or a still non-stabilized genome within each cell line.

aCGH were then performed (using controlled 8MGBA cells as the reference) to analyze which regions were amplified or deleted **(data not shown).** Data revealed that all αS-SETMAR-8MGBA chromosomes were affected, confirming a global mechanism of chromosomal chaos which affected not only the number of chromosomes, but also their organization. These data do suggest a variety of mechanisms, promoted by αSETMAR at the root of such chaos, i.e. both aneuploidy and chromosomal rearrangement.

Aneuploidy is one of the most striking genomic abnormalities in cancer. We thus assumed that αS-SETMAR impaired normal chromosome disjunction during mitosis. As previously shown **(data not shwon),** 40 genes involved either in spindle assembly or chromosomes segregation were deregulated by αS-SETMAR. By removing the duplicates within these lists, we obtained a list of 35 genes (S4A Table), representing 53% of the 66 cell cycle DEG **(data not shwon),** that may control this key stage of mitosis through two main pathways, spindle assembly and centrosomes behavior. The final list contained 31 genes, including some previously discussed (PLK2, RGCC and TTN). Among the 31 genes, the chaos caused by αS-SETMAR overexpression has led to the modification in genes number for 17 ones (about the half), for which αS-SETMAR-8MGBA cells contained a different number of copies than the 8MGBA parental cell line. For 8 of them, it was difficult to state that the LogFC variation in RNA-seq was due to αS-SETMAR rather than to the change in gene copies number, because variations were positively correlated between RNA-seq and aCGH analysis. For the other 23, we assumed that variation in RNA-seq was only due to αS-SETMAR overexpression. Among them, 19 were directly involved either in spindle assembly or centrosome functions (data not shown). In a recent paper, Samejima et al [34] have reviewed and proposed a list of the 367 main centromeric proteins, in which 11 of our 19 DEG were present **(data not shown).** During αS-SETMAR-8MGBA cell production, this probably caused a mis-regulation of chromosomes segregation, leading to aneuploid cell lines. It should also be remembered that any SETMAR variant with an intact MAR domain can bind to centromeres via CENP-B-like sequences, which resemble TIRs [13]. Thus, the aneuploidy observed following αS-SETMAR overexpression could be due to deregulation of genes encoding proteins involved in spindle organization and chromosome segregation, but also by a direct effect of αS-SETMAR at the centromeres. Both hypotheses are not mutually exclusive, highlighting a new role for SETMAR, in particular the short one.

In addition to aneuploidy, aCGH revealed that αS-SETMAR-8MGBA cells have undergone a major reorganization of chromosomes structure, leading to "chromosomal chaos". Data presented here **(data not shown)** suggested two kinds of rearrangement, i.e. translocations and genes copy number variations. For the last fifteen years, catastrophic events causing multiple complex chromosomal rearrangements, sometimes referred to as chromoanagenesis, have been proposed to occur at one or more loci during the cell cycle. Some events depend on disorders during the replication step while others occur upon mitosis, two stages greatly affected by αS-SETMAR over-expression. Translocations involving more than two chromosomes are known as chromoplexy [35] and are not associated with changes in gene copies number. In contrast, chromoanasynthesis and chromothripsis lead to a variation in gene copies number. In chromoanasynthesis, defects during DNA replication promotes amplifications (duplications/triplications) and deletions of large regions on the rearranged chromosome. In contrast, during chromothripsis, that takes place upon mitosis, a chromosome arm is destroyed, producing many DNA fragments, before incomplete and accidental restoration. Upon restoration, orientation of the fragments can change, some are removed and others may form extrachromosomal double min. In this case, no gain in gene copies number is expected. However, this research area is still evolving, and some authors consider it possible that chromothripsis may be associated with a moderate increase in copies number (two to three)[36]. These massive genomic rearrangements are believed to be generated in a single catastrophic event [37]. They change our traditional view of tumorigenesis as a gradual process of the mutations accumulation, providing the rapid accrual of hundreds of rearrangements within a few cell divisions. More, chromothripsis seems to occur more frequently than initially thought, with a frequency of more than 50% in several cancer types, including GB [36]. Finally, repeated sequences have often been suggested as the source of such changes. We should point out here that no relationship could be established between the areas of chromosomal rearrangement (seen in aCGH) and Hsmar1 TIR. According to what was observed in S-SETMAR-8MGBA karyotypes, we assume that the 8MGBA cells underwent chromoanagenesis during the course of their transformation by αS-SETMAR, with, at the same time, a mixture of various chromosomal events. During these events, the precise role of αS-SETMAR and its DEG (like PLK2, TTN and RGGC) remains to be established and opens up a new way of understanding the mechanisms involved.

### aS-SETMAR and cell death

One point that remains intriguing for the scientific community interested in chromosomal chaos is how cells can survive such crises, which normally must be resolved by cell death, either by apoptosis or autophagy. Such cell fate was in fact noted when analyzing the time-lapse motions, where we can see, for αS-SETMAR-8MGBA (but not for 8MGBA), that some cells did not pass through the mitosis stage. They appeared to be blocked, sometimes for several hours (about 21 for the two cells marked data not shown) and then disintegrated. We thus looked to what extent both cell lines triggered apoptosis and/or autophagy, in standard growing conditions or after induction.

**Apoptosis.** In the absence of apoptosis induction, the level of cleaved PARP (PARPc, an apoptosis marker) (**Fig 4A** revealed no differences between control 8MGBA cells and those over-expressing αS-SETMAR, with no signal for both cells. However, results were different if cells were beforehand treated with 1 µM doxorubicin for 48 hours to induce a high level of DNA double strand breaks (i.e. these conditions differed from those used during irradiation, where cells suffered DNA double strand breaks level that triggers repair mechanisms not apoptosis). Here, the PARPc signal was significantly higher (p=0.0025) in αS-SETMAR-8MGBA cells when compared to 8MGBA cells **(****Fig 4A****).**

From the RNA-seq data we already generated, we found that apoptosis was moderately affected by αS-SETMAR over-expression, since only 13 genes were concerned **(data not shown),** with 6 up-regulated (including PUMA) and 7 down-regulated. These results were in line with those previously published, apoptosis having never been identified as a main target for FL-SETMAR. For 4 up-regulated genes (including PUMA), the effect can be attributed to an increase in the number of copies of the gene in αS-SETMAR-8MGBA cells, rather than to a transcriptional effect of αS-SETMAR. Finally, only 9 genes involved in apoptosis appeared to be deregulated by αS-SETMAR overexpression, probably accounting for the increased response of αS-SETMAR-8MGBA cells to doxorubicin treatment.

**Autophagy.** To test the sensitivity of αS-SETMAR-8MGBA and 8MGBA cells to autophagy, both cell lines were deprived of serum for 3 days. This treatment impaired down the proliferation of the two cell lines by a factor of 7.5 and 10 for 8MGBA and αS-SETMAR-8MGBA respectively, the difference being significant **(****Fig 4B****).**

From the RNA-seq data we already generated, we identified 46 DEG for this mechanism **(data not shown).** After eliminating those which expression can be deregulated by a variation in the gene copies number, we retained 14 positively DEG and 9 negatively DEG. Among them, ULK1, a major gene in autophagy regulation, was overexpressed in αS-SETMAR-8MGBA (LogFC=1.69802). We also noted that two other main genes (RFPL2 and DEPTOR) can be directly upregulated by αS-SETMAR since they had either a TIR within their sequence (DEPTOR) or were targeted by S-SETMAR in ChIP-seq analysis (RFPL2 [10]).

The aim of this work was to understand how αS-SETMAR could play a protective role in GB. Analysis of recombinant cell lines overexpressing αS-SETMAR highlighted that this short variant reduces cell proliferation by slowing the cycle duration by a factor of 1.37 (from 27 to 37 hours) and makes cells more sensitive to stringent conditions for their survival, such as those induced by the radio- and/or chemotherapy administered to patients.

Unexpectedly, our study highlighted that αS-SETMAR overexpression creates chromosomal chaos, both in terms of chromosomes number (aneuploidy) and organization (chromoanagenesis). We identified 19 candidate genes, all of which may be responsible for such chaos. They are directly involved either in spindle assembly and/or centrosome functions.

We propose that this chromosomal chaos is responsible for αS-SETMAR-8MGBA cells greater fragility, greater sensitivity to cell death induction, whether by apoptosis or autophagy.

### REFERENCES:

Throughout this application, various references describe the state of the art to which this invention pertains. The disclosures of these references are hereby incorporated by reference into the present disclosure.
1. Ostrom QT, Cioffi G, Gittleman H, Patil N, Waite K, Kruchko C, et al. CBTRUS Statistical Report: Primary Brain and Other Central Nervous System Tumors Diagnosed in the United States in 2012-2016. Neuro-oncology. 2019;21: v1-v100. doi:10.1093/neuonc/noz150
2. Friedmann-Morvinski D. Glioblastoma heterogeneity and cancer cell plasticity. Crit Rev Oncog. 2014;19: 327-336. doi:10.1615/critrevoncog.2014011777
3. Lié O, Virolle T, Gabut M, Pasquier C, Zemmoura I, Augé-Gouillou C. SETMAR Shorter Isoform: A New Prognostic Factor in Glioblastoma. Front Oncol. 2021;11: 638397. doi:10.3389/fonc.2021.638397
4. Lié O, Renault S, Augé-Gouillou C. SETMAR, a case of primate co-opted genes: towards new perspectives. Mob DNA. 2022;13: 9. doi:10.1186/s13100-022-00267-1
5. Carlson SM, Moore KE, Sankaran SM, Elias JE, Gozani O. A Proteomic Strategy Identifies Lysine Methylation of Splicing Factor snRNP70 by SETMAR. J Biol Chem. 2015. doi:10.1074/jbc.M115.641530
6. De Haro LP, Wray J, Williamson EA, Durant ST, Corwin L, Gentry AC, et al. Metnase promotes restart and repair of stalled and collapsed replication forks. Nucleic Acids Res. 2010;38: 5681-5691. doi:10.1093/nar/gkq339
7. Wray J, Williamson EA, Sheema S, Lee S-H, Libby E, Willman CL, et al. Metnase mediates chromosome decatenation in acute leukemia cells. Blood. 2009;114: 1852-1858. doi:10.1182/blood-2008-08-175760
8. Tellier M, Chalmers R. The roles of the human SETMAR (Metnase) protein in illegitimate DNA recombination and non-homologous end joining repair. DNA Repair (Amst). 2019;80: 26-35. doi:10.1016/j.dnarep.2019.06.006
9. Dussaussois-Montagne A, Jaillet J, Babin L, Verrelle P, Karayan-Tapon L, Renault S, et al. SETMAR isoforms in glioblastoma: A matter of protein stability. Oncotarget. 2017;8: 9835-9848. doi:10.18632/oncotarget.14218
10. Antoine-Lorquin A, Arensburger P, Arnaoty A, Asgari S, Batailler M, Beauclair L, et al. Two repeated motifs enriched within some enhancers and origins of replication are bound by SETMAR isoforms in human colon cells. Genomics. 2021;113: 1589-1604. doi:10.1016/j.ygeno.2021.03.032
11. Xie R, Chen X, Cheng L, Huang M, Zhou Q, Zhang J, et al. NONO Inhibits Lymphatic Metastasis of Bladder Cancer via Alternative Splicing of SETMAR. Mol Ther. 2020. doi:10.1016/j.ymthe.2020.08.018
12. Jeyaratnam DC, Baduin BS, Hansen MC, Hansen M, Jorgensen JM, Aggerholm A, et al. Delineation of Known and New Transcript Variants of the SETMAR (Metnase) Gene and the Expression Profile in Hematological Neoplasms. Experimental Hematology. [cited 5 May 2014]. doi:10.1016/j.exphem.2014.02.005
13. Tellier M, Chalmers R. Human SETMAR is a DNA sequence-specific histonemethylase with a broad effect on the transcriptome. Nucleic Acids Res. 2019;47: 122-133. doi:10.1093/nar/gky937
14. Williamson EA, Rasila KK, Corwin LK, Wray J, Beck BD, Severns V, et al. The SET and transposase domain protein Metnase enhances chromosome decatenation: regulation by automethylation. Nucleic Acids Res. 2008;36: 5822-5831. doi:10.1093/nar/gkn560
15. Miskei M, Horváth A, Viola L, Varga L, Nagy É, Feró O, et al. Genome-wide mapping of binding sites of the transposase-derived SETMAR protein in the human genome. Comput Struct Biotechnol J. 2021;19: 4032-4041. doi:10.1016/j.csbj.2021.07.010
16. Chen Q, Bates AM, Hanquier JN, Simpson E, Rusch DB, Podicheti R, et al. Structural and genome-wide analyses suggest that transposon-derived protein SETMAR alters transcription and splicing. JBiol Chem. 2022;298: 101894. doi:10.1016/j.jbc.2022.101894
17. Schneider CA, Rasband WS, Eliceiri KW. NIH Image to ImageJ: 25 years of image analysis. Nat Methods. 2012;9: 671-675. doi:10.1038/nmeth.2089
18. Caldon CE, Burgess A. Label free, quantitative single-cell fate tracking of time-lapse movies. MethodsX. 2019;6: 2468-2475. doi:10.1016/j.mex.2019.10.014
19. Sharma A, Singh K, Almasan A. Histone H2AX phosphorylation: a marker for DNA damage. Methods Mol Biol. 2012;920: 613-626. doi:10.1007/978-1-61779-998-3_40
20. Tiek DM, Rone JD, Graham GT, Pannkuk EL, Haddad BR, Riggins RB. Alterations in Cell Motility, Proliferation, and Metabolism in Novel Models of Acquired Temozolomide Resistant Glioblastoma. Sci Rep. 2018;8: 7222. doi:10.1038/s41598-018-25588-1
21. Chen R, Chen C, Han N, Guo W, Deng H, Wang Y, et al. Annexin-1 is an oncogene in glioblastoma and causes tumour immune escape through the indirect upregulation of interleukin-8. J Cell Mol Med. 2022;26: 4343-4356. doi:10.1111/jcmm.17458
22. Pibuel MA, Poodts D, Diaz M, Hajos SE, Lompardía SL. The scrambled story between hyaluronan and glioblastoma. J Biol Chem. 2021;296: 100549. doi: 10.1016/j .jbc.2021.100549
23. Xia X, Cao F, Yuan X, Zhang Q, Chen W, Yu Y, et al. Low expression or hypermethylation of PLK2 might predict favorable prognosis for patients with glioblastoma multiforme. PeerJ. 2019;7: e7974. doi:10.7717/peerj.7974
24. Nadjar Y, Triller A, Bessereau J-L, Dumoulin A. The Susd2 protein regulates neurite growth and excitatory synaptic density in hippocampal cultures. Mol Cell Neurosci. 2015;65: 82-91. doi:10.1016/j.mcn.2015.02.007
25. Pan W, Cheng Y, Zhang H, Liu B, Mo X, Li T, et al. CSBF/C10orf99, a novel potential cytokine, inhibits colon cancer cell growth through inducing G1 arrest. Sci Rep. 2014;4: 6812. doi:10.1038/srep06812
26. Guo Z, Chen M, Chao Y, Cai C, Liu L, Zhao L, et al. RGCC balances selfrenewal and neuronal differentiation of neural stem cells in the developing mammalian neocortex. EMBORep. 2021;22: e51781. doi:10.15252/embr.202051781
27. Tatomir A, Cuevas J, Badea TC, Muresanu DF, Rus V, Rus H. Role of RGC-32 in multiple sclerosis and neuroinflammation - few answers and many questions. Front Immunol. 2022;13: 979414. doi:10.3389/fimmu.2022.979414
28. Saigusa K, Imoto I, Tanikawa C, Aoyagi M, Ohno K, Nakamura Y, et al. RGC32, a novel p53-inducible gene, is located on centrosomes during mitosis and results in G2/M arrest. Oncogene. 2007;26: 1110-1121. doi:10.1038/sj.onc.1210148
29. Bredel M, Bredel C, Juric D, Duran GE, Yu RX, Harsh GR, et al. Tumor necrosis factor-alpha-induced protein 3 as a putative regulator of nuclear factor-kappaB-mediated resistance to O6-alkylating agents in human glioblastomas. J Clin Oncol. 2006;24: 274-287. doi:10.1200/JCO.2005.02.9405
30. Vlaicu SI, Tatomir A, Rus V, Rus H. Role of C5b-9 and RGC-32 in Cancer. Front Immunol. 2019;10: 1054. doi:10.3389/fimmu.2019.01054
31. Fankhaenel M, Hashemi FSG, Mourao L, Lucas E, Hosawi MM, Skipp P, et al. Annexin A1 is a polarity cue that directs mitotic spindle orientation during mammalian epithelial morphogenesis. Nat Commun. 2023;14: 151. doi:10.1038/s41467-023-35881-x
32. Zhang C, Ni C, Lu H. Polo-Like Kinase 2: From Principle to Practice. Front Oncol. 2022;12: 956225. doi:10.3389/fonc.2022.956225
33. Zhong Z, Wilson KL, Dahl KN. Beyond lamins other structural components of the nucleoskeleton. Methods Cell Biol. 2010;98: 97-119. doi:10.1016/S0091-679X(10)98005-9
34. Samejima I, Platani M, Earnshaw WC. Use of Mass Spectrometry to Study the Centromere and Kinetochore. Prog Mol Subcell Biol. 2017;56: 3-27. doi:10.1007/978-3-319-58592-5_1
35. Shorokhova M, Nikolsky N, Grinchuk T. Chromothripsis-Explosion in Genetic Science. Cells. 2021;10: 1102. doi:10.3390/cells10051102
36. Cortés-Ciriano I, Lee JJ-K, Xi R, Jain D, Jung YL, Yang L, et al. Comprehensive analysis of chromothripsis in 2,658 human cancers using whole-genome sequencing. Nat Genet. 2020;52: 331-341. doi:10.1038/s41588-019-0576-7
37. Stephens PJ, Greenman CD, Fu B, Yang F, Bignell GR, Mudie LJ, et al. Massive genomic rearrangement acquired in a single catastrophic event during cancer development. Cell. 2011;144: 27-40. doi:10.1016/j.cell.2010.11.055
38. Cimini D, Howell B, Maddox P, Khodjakov A, Degrassi F, Salmon ED. Merotelic kinetochore orientation is a major mechanism of aneuploidy in mitotic mammalian tissue cells. J Cell Biol. 2001;153: 517-527. doi:10.1083/jcb.153.3.517
39. Sazonova EV, Petrichuk SV, Kopeina GS, Zhivotovsky B. A link between mitotic defects and mitotic catastrophe: detection and cell fate. Biol Direct. 2021;16: 25. doi:10.1186/s 13062-021-00313-7

## Claims

1. A method for sensitizing glioblastoma (GB) cells to a classical treatment comprising a step of administrating an agonist of αS-SETMAR in a subject in need thereof.

2. The method for sensitizing GB cells according to claim 1 wherein, the classical treatment is selected from the group consisting of but not limited to: targeted therapy, radiation therapy, immunotherapy, chemotherapy (temozolomide (TMZ)) or surgery.

3. The method for sensitizing GB cells according to claim 1 wherein, the agonist of αS-SETMAR is selected from the group consisting of but not limited to : polypeptide, nucleic acid, mRNA or ASO.

4. The method for sensitizing GB cells according to claim 3 wherein, the agonist of αS-SETMAR is delivered by ultrasound.

5. The method for sensitizing GB cells according to claim 4 wherein, the agonist of αS-SETMAR is obtained by exon skipping.

6. i)an agonist of αS-SETMAR and ii) a classical treatment for use by simultaneous, separate or sequential administration in the prevention and/or treatment of GB and/or metastatic cancer in a subject in need thereof.

7. A pharmaceutical composition which comprises the αS-SETMAR agonist.

8. The pharmaceutical composition comprising αS-SETMAR agonist for treating glioblastoma.
